# EUROPEAN PATENT APPLICATION

(11) **EP 4 588 479 A1**
(43) Date of publication of application: **23.07.2025**
(21) Application number: 23864543.6
(22) Date of filing: 18.08.2023
(51) Int. Cl.: A61K 31/661, A61K 31/6615, A61K 31/664, A61K 31/675, A61K 31/683, C07F 9/09, C07F 9/6533, C07F 9/24, C07F 9/12, A61P 3/10, A61P 3/04, A61P 3/00, A61P 9/10, A61P 35/00, A61P 25/16, A61P 25/28, A61P 1/16

(54) **USE OF 6-PHOSPHOGLUCONIC ACID AND DERIVATIVE THEREOF IN PREPARING MEDICAMENT FOR PREVENTING OR TREATING GLYCOMETABOLISM DISORDER DISEASES**

(30) Priority: 16.09.2022 CN 202211126387
(71) Applicant: Fudan University, Shanghai 200433 (CN)
(72) Inventor: ZHOU, Lu, Shanghai 200433 (CN); LI, Jin, Shanghai 200433 (CN); HUANG, He, Shanghai 200433 (CN); XIE, Wenhao, Shanghai 200433 (CN); ZHANG, Yufeng, Shanghai 200433 (CN); GAO, Wei, Shanghai 200433 (CN)
(74) Representative: Zaboliene, Reda
(86) International application number: PCT/CN2023/113692
(87) International publication number: WO 2024/055810

(57) **Abstract**

Use of 6-phosphogluconic acid and a derivative thereof in preparing a medicament for preventing or treating glycometabolism disorder diseases. The 6-phosphogluconic acid and the derivative thereof are a type of cell glycometabolism regulators and diabetes treatment agents, have a remarkable regulation effect on islet alpha cells, and can be further prepared into a medicament for preventing or treating diabetes and other glycometabolism disorder diseases.

## Description

### TECHNICAL FIELD

The present disclosure relates to pharmaceutical science, and more particularly, to the use of 6-phosphogluconic acid and derivatives thereof in preparing medicaments for preventing or treating glucose metabolism disorder diseases.

### BACKGROUND

Diabetes is a syndrome of sugar, fat, and protein metabolism disorders caused by an absolute or relative lack of insulin secretion or insulin utilization impairment (i.e., insulin resistance). The main signs of diabetes are hyperglycemia, impaired glucose metabolism, and diabetic urine. According to the cause and pathological mechanism, diabetes is mainly divided into type 1 diabetes mellitus, type 2 diabetes mellitus, other specific types of diabetes and gestational diabetes. Type 1 diabetes mellitus and type 2 diabetes mellitus are clinically common. Type 1 diabetes mellitus, also known as insulin-dependent diabetes, is believed to be caused by the absolute insufficiency of insulin secretion due to the destruction of beta cells in the islets due to autoimmunity. Type 2 diabetes mellitus, also known as non-insulin-dependent diabetes, is believed to be caused by a relative insufficiency of insulin secretion characterized by lack of insulin receptors and decreased sensitivity, insulin resistance and utilization disorders.

The pentose phosphate pathway (PPP) is another type of sugar metabolism pathway in organisms other than anaerobic glycolysis and aerobic oxidation, which can be divided into two stages. The first stage is an oxidation reaction, starting with the dehydrogenation of glucose-6-phosphate catalyzed by glucose-6-phosphate dehydrogenase to generate 6-phosphoglucono-δ-lactone. The 6-phosphoglucono-δ-lactone is hydrolyzed to 6-phosphogluconic acid under the action of 6-posphogluconolactonase. And then, the 6-phosphogluconic acid re-hydrogenated under the action of 6-phosphogluconate dehydrogenase and spontaneously decarboxylation to ribulose-5-phosphate, while generating nicotinamide adenine dinucleotide phosphate (NADPH) and CO₂. The second phase is a non-oxidation reaction, wherein the ribulose-5-phosphate is converted into fructose-6-phosphate and glyceraldehyde-3-phosphate through a series of group transfer reactions to enter the glycolysis pathway.

6-phosphogluconic acid is a key metabolite intermediate in the PPP pathway, which is obtained through phosphorylation of glucose by hexokinase, oxidative dehydrogenation by glucose-6-phosphate dehydrogenase and hydrolysis by 6-phosphoglucolactonase. It can further be oxidatively decarboxylated by 6-phosphogluconate dehydrogenase to generate ribulose-5-phosphate. The ribulose-5-phosphate undergoes a series of transketolase or transaldolase reactions and isomerizes through metabolic intermediates such as erythrose 4-phosphate, pentose phosphate, and heptose phosphate to generate glyceraldehyde-3-phosphate and fructose-6-phosphate and re-enter the glycolysis path. As a key metabolic intermediate in the glucose metabolism pathway, only a few reports have observed reduced levels of 6-phosphogluconic acid in the red blood cells of patients with type II diabetes (J.Clin.Med.2020; doi:10.3390/jcm9061619). However, since the polarity of 6-phosphogluconic acid is too large to penetrate cell membranes and enter cells, there is no research report that 6-phosphogluconic acid is used as a regulator to be applied to regulation of endogenous sugar metabolism pathways, and there is no further study on structural modification and molecular optimization of 6-phosphogluconic acid for endogenous sugar metabolism pathway, and no research reports that 6-phosphogluconic acid and similar compounds thereof are used as modulators of endogenous sugar metabolism pathways to treat diabetes.

### BRIEF SUMMARY

A first objective of the present disclosure is to provide a use of 6-phosphogluconic acid and derivatives thereof, or pharmaceutically acceptable salts thereof, in preparing medicament for preventing or treating glucose metabolism disorder diseases. The 6-phosphogluconic acid and the derivatives thereof have a remarkable regulation effect on the insulin secretion function of islet α-cells and can be regulators for endogenous glucose metabolism pathways in treating glucose metabolism disorder diseases.

A second objective of the present disclosure is to provide derivatives of 6-phosphogluconic acid. The derivatives obtained by modification of 6-phosphogluconic acid are able to penetrate cell membranes and enter cells directly, where are absorbed by islet α-cells and releases 6-phosphogluconic acid, so that the expression of insulin secretion related genes in islet α-cells is improved and the insulin secretion of islet α-cells is stimulated.

A third objective of the present disclosure is to provide a pharmaceutical composition, in which 6-phosphogluconic acid or derivatives or pharmaceutically acceptable salts thereof is delivered into cells by a pharmaceutically acceptable drug delivery vehicle and absorbed by islet α-cells, so that the expression of insulin secretion related genes in islet α-cells is improved and the insulin secretion of islet α-cells is stimulated.

A fourth objective of the present disclosure is to provide a use of the pharmaceutical composition in preparing medicament for preventing or treating glucose metabolism disorder diseases, wherein the glucose metabolism disorder diseases include diabetes, diabetic ketoacidosis, hyperosmolar hyperglycemic nonketotic coma, diabetic retinopathy, diabetic nephropathy, diabetic foot, atherosclerosis, cerebrovascular accident, peripheral vascular disease, fatty liver disease, malignant tumor, Alzheimer's disease and Parkinson's disease.

To achieve the first objective above-mentioned, the present disclosure provides a use of 6-phosphogluconic acid and derivatives thereof, or pharmaceutically acceptable salts thereof, in preparing medicament for preventing or treating glucose metabolism disorder diseases.

As a preferred embodiment, the derivative of the 6-phosphogluconic acid is comprising one or more modified terminal phosphate groups including phosphate esters, phosphines, phosphorane ylides, phosphates, aminophosphate esters, phosphonates, hypophosphites, phosphine oxides, thiophosphates, fluorophosphates, phosphoric anhydrides, bisphosphonates, phosphite esters, phosphonites, thiophosphite esters, dithiophosphate esters and phosphonamides;
or a derivative comprising one or more modified terminal carboxylic acid groups including carboxylic esters, aminocarboxylic esters, amides and thiocarbamate esters,
or a derivative comprising one or more modified hydroxyl groups on a sugar chain, which includes substituted hydroxyls, hydroxy esters, hydroxy amides, thiols, substituted thiols and thioesters;
or a derivative in which each structural fragment is modified by a combination of groups mentioned above.

As a preferred embodiment, the derivative of the 6-phosphogluconic acid is represented by Formula (I), (II), (III), (IV) or (V), 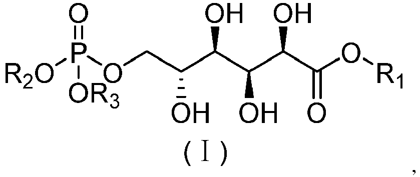 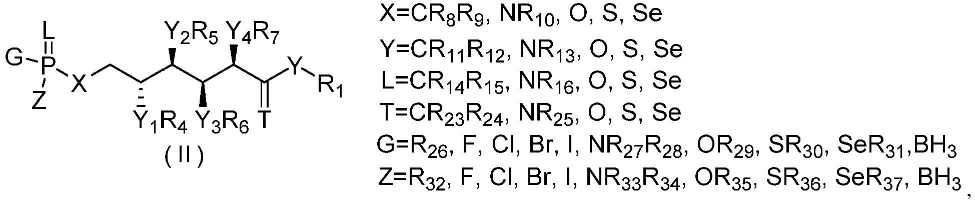 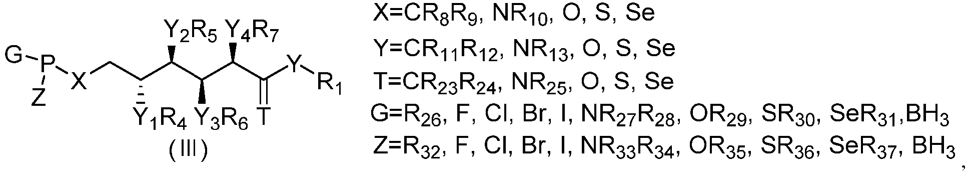 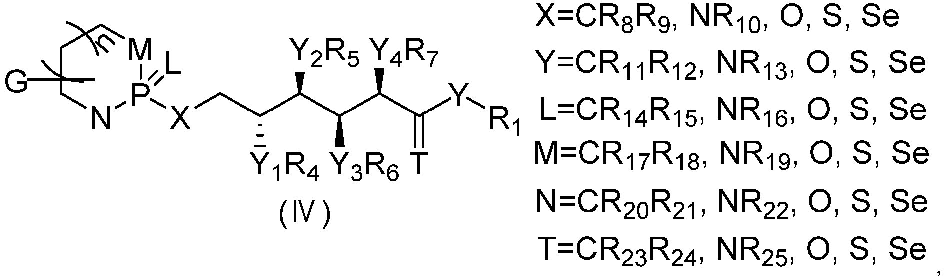 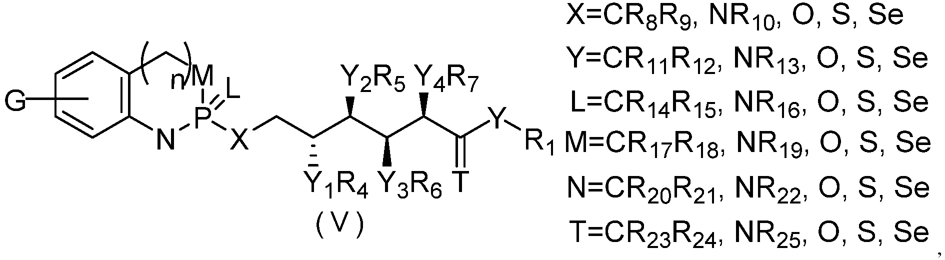 wherein,
R₁₋₃₇ are selected from hydrogen atom, alkyl or substituted alkyl containing less than 10 carbon atoms, cycloalkyl or substituted cycloalkyl, alkylaryl or substituted alkylaryl, alkenyl or substituted alkenyl containing less than 10 carbon atoms, cycloalkenyl or substituted cycloalkenyl, alkenylaryl or substituted alkenylaryl, alkynyl or substituted alkynyl containing less than 10 carbon atoms, alkynylaryl or substituted alkynylaryl containing less than 10 carbon atoms; or selected from biomolecular fragments including oligopeptide, glycoside, protein, nucleotide and fatty acid; or combinations of the above groups and fragments; and wherein a substituent is selected from halogens and heteroatoms.

In the present disclosure, Y₁, Y₂, Y₃ and Y₄ in the Formula (II), (III), (IV) or (V) are each, independently of one another, selected from the Y fragment. In the Formula (II), (III), (IV) or (V), X represents one of CR₈R₉, NR₁₀, O, S and Se; Y represents one of CR₁₁R₁₂, NR₁₃, O, S and Se; L represents one of CR₁₄R₁₅, NR₁₆, O, S and Se; M represents one of CR₁₇R₁₈, NR₁₉, O, S and Se; N represents one of CR₂₀R₂₁, NR₂₂, O, S and Se; T represents one of CR₂₃R₂₄, NR₂₅, O, S and Se; G represents one of R₂₆, F, Cl, Br, I, NR₂₇R₂₈, OR₂₉, SR₃₀, SeR₃₁ and BH₃; and, Z represents one of R₃₂, F, Cl, Br, I, NR₃₃R₃₄, OR₃₅, SR₃₆, SeR₃₇ and BH₃. In the Formula (IV) or (V), n represents the length of the carbon chain at the marked position, and the value of n is 0, 1 or 2.

As a preferred embodiment, the glucose metabolism disorder diseases include diabetes, diabetic ketoacidosis, hyperosmolar hyperglycemic nonketotic coma, diabetic retinopathy, diabetic nephropathy, diabetic foot, atherosclerosis, cerebrovascular accident, peripheral vascular disease, fatty liver disease, malignant tumor, Alzheimer's disease and Parkinson's disease.

To achieve the above-mentioned second objective, the present disclosure provides a derivative of 6-phosphogluconic acid. The derivative of the 6-phosphogluconic acid is represented by Formula (I), (II), (III), (IV) or (V), 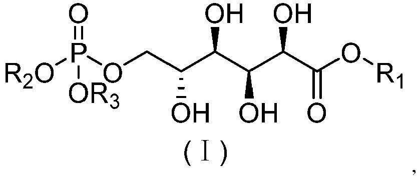 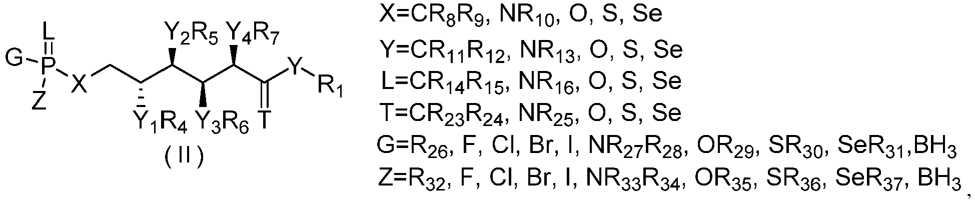 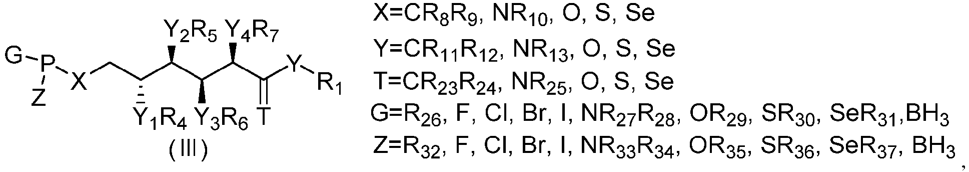 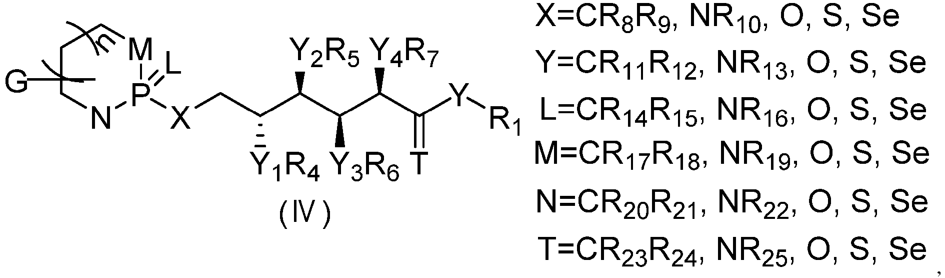 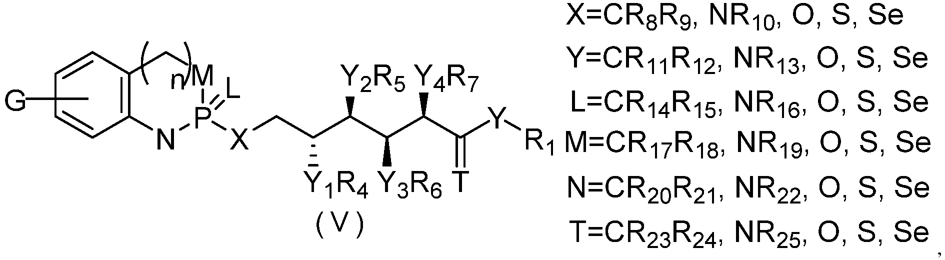 wherein,
R₁₋₃₇ are selected from hydrogen atom, alkyl or substituted alkyl containing less than 10 carbon atoms, cycloalkyl or substituted cycloalkyl, alkylaryl or substituted alkylaryl, alkenyl or substituted alkenyl containing less than 10 carbon atoms, cycloalkenyl or substituted cycloalkenyl, alkenylaryl or substituted alkenylaryl, alkynyl or substituted alkynyl containing less than 10 carbon atoms, alkynylaryl or substituted alkynylaryl containing less than 10 carbon atoms; or selected from biomolecular fragments including oligopeptide, glycoside, protein, nucleotide and fatty acid; or combinations of the above groups and fragments; and wherein a substituent is selected from halogens and heteroatoms.

To achieve the above-mentioned third objective, the present disclosure provides a pharmaceutical composition. The pharmaceutical composition comprises 6-phosphogluconic acid or derivatives or pharmaceutically acceptable salts thereof, and a pharmaceutically acceptable drug delivery vehicle, wherein the pharmaceutical composition is modified by the drug delivery vehicle to penetrate cell membranes.

As a preferred embodiment, the derivative of the 6-phosphogluconic acid is a derivative comprising one or more modified terminal phosphate groups including phosphate ester, phosphine, phosphorane ylide, phosphate, aminophosphate ester, phosphonate, hypophosphite, phosphine oxide, thiophosphate, fluorophosphate, phosphoric anhydride, bisphosphonate, phosphite ester, phosphonite, thiophosphite ester, dithiophosphate ester and phosphonamide;
or a derivative comprising one or more modified terminal carboxylic acid groups including carboxylic esters, aminocarboxylic esters, amides and thiocarbamate esters;
or a derivative comprising one or more modified hydroxyl groups on a sugar chain, which includes substituted hydroxyls, hydroxy esters, hydroxy amides, thiols, substituted thiols and thioesters;
or a derivative in which each structural fragment is modified by a combination of groups mentioned above.

As a preferred embodiment, the derivative of the 6-phosphogluconic acid is represented by Formula (I), (II), (III), (IV) or (V), 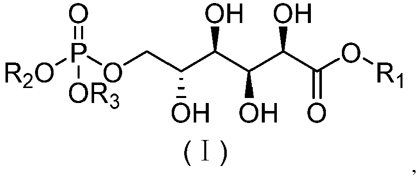 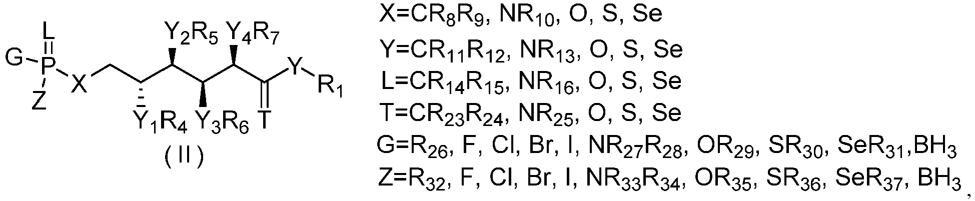 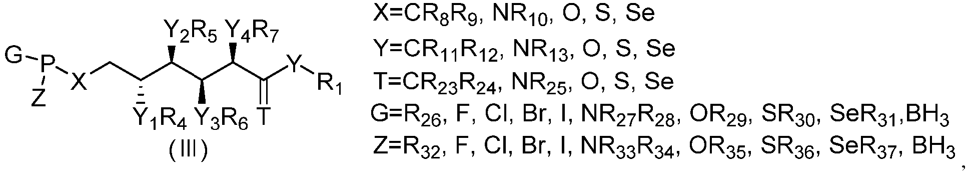 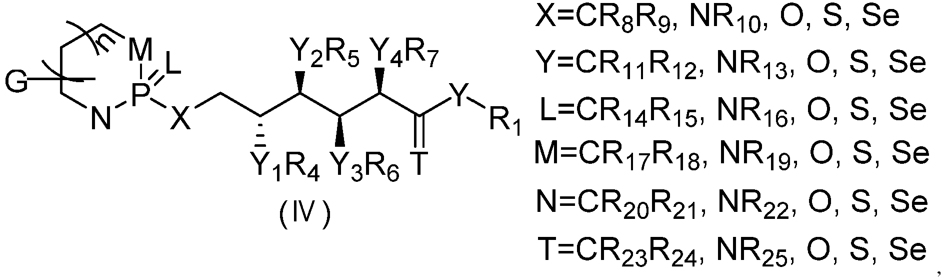 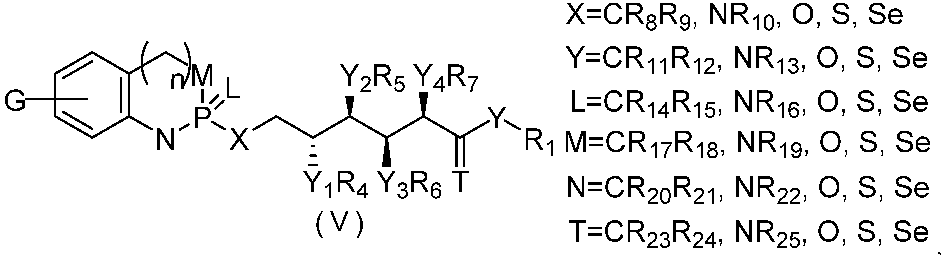 wherein,
R₁₋₃₇ are selected from hydrogen atom, alkyl or substituted alkyl containing less than 10 carbon atoms, cycloalkyl or substituted cycloalkyl, alkylaryl or substituted alkylaryl, alkenyl or substituted alkenyl containing less than 10 carbon atoms, cycloalkenyl or substituted cycloalkenyl, alkenylaryl or substituted alkenylaryl, alkynyl or substituted alkynyl containing less than 10 carbon atoms, alkynylaryl or substituted alkynylaryl containing less than 10 carbon atoms; or selected from biomolecular fragments including oligopeptide, glycoside, protein, nucleotide and fatty acid; or combinations of the above groups and fragments; and wherein a substituent is selected from halogens and heteroatoms.

As a preferred embodiment, the drug delivery vehicle is a nano drug delivery system including liposomes, polymeric micelles or microcapsules, lipid nanoparticles, and chitosan nanoparticles, wherein the 6-phosphogluconic acid or derivatives or pharmaceutically acceptable salts thereof are encapsulated in the drug delivery vehicle.

To achieve the above-mentioned fourth objective, the present disclosure provides a use of the pharmaceutical composition in preparing medicament for preventing or treating glucose metabolism disorder diseases. The glucose metabolism disorder diseases include diabetes, diabetic ketoacidosis, hyperosmolar hyperglycemic nonketotic coma, diabetic retinopathy, diabetic nephropathy, diabetic foot, atherosclerosis, cerebrovascular accident, peripheral vascular disease, fatty liver disease, malignant tumor, Alzheimer's disease and Parkinson's disease.

It has been verified by experiments that a preparation of 6-phosphogluconic acid encapsulated by the drug delivery vehicle, the carboxylate derivatives and/or phosphate derivatives of 6-phosphogluconic acid, a preparation of carboxylate derivatives and/or phosphate derivatives of 6-phosphogluconic acid encapsulated by the drug delivery vehicle, a preparation of 6-phosphogluconic acid salt encapsulated by the drug delivery vehicle, salts of the carboxylate derivatives and/or phosphate derivatives of 6-phosphogluconic acid, and a preparation of the salts of the carboxylate derivatives and/or phosphate derivatives of 6-phosphogluconic acid encapsulated by the drug delivery vehicle have an increased penetration rate, which may penetrate cell membranes to directly enter cells and be absorbed by islet α-cells while release 6-phosphogluconic acid, so that the expression of insulin secretion related genes in islet α-cells is improved and the insulin secretion of islet α-cells is stimulated.

The drug delivery vehicle is a nano drug delivery system of drug compound/composition solutions or suspensions, in which the 6-phosphogluconic acid or derivatives or pharmaceutically acceptable salts thereof are encapsulated.

The basis of the present disclosure lies in the discovery made through metabolomics research, which revealed that the content of 6-phosphogluconic acid is significantly lower in human islet α-cells compared to islet β-cells. After exogenous supplementation of 6-phosphogluconic acid or the derivatives thereof, islet α-cells exhibited high insulin expression levels. Q-PCR detection revealed a significant increase in the transcription level of insulin synthesis-related genes in islet α-cells treated with 6-phosphogluconic acid or the derivatives thereof, indicating β-cells-like functionalization. 6-Phosphogluconic acid cannot penetrate the cell membranes and be absorbed by islet α-cells due to its large polar groups, such as carboxylate groups and phosphate groups. Therefore, a cell membrane permeable composition containing 6-phosphogluconic acid or pharmaceutically acceptable salts thereof and a pharmaceutically acceptable drug delivery vehicle, as well as derivatives of 6-phosphogluconic acid which is obtained by modifying the chemical structure of 6-phosphogluconic acid and serves as a precursor of 6-phosphogluconic acid, may penetrate cell membranes to enter cells directly, and then be absorbed by islet α-cells while release 6-phosphogluconic acid, so that the intracellular concentration of 6-phosphogluconic acid is increased. The expression of insulin secretion related genes in islet α-cells is improved and the insulin secretion of islet α-cells is stimulated.

In embodiments of the present disclosure, derivatives of the following structural formulas are prepared, of which the biological activity is verified. Both of the carboxylate derivatives and/or phosphate derivatives of 6-phosphogluconic acid can be absorbed by islet α-cells and release 6-phosphogluconic acid.

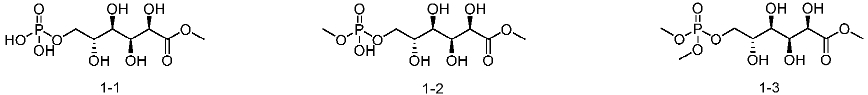

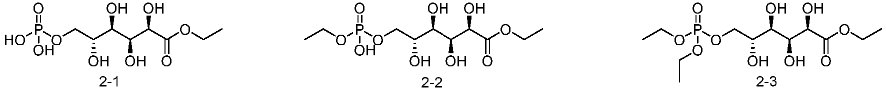

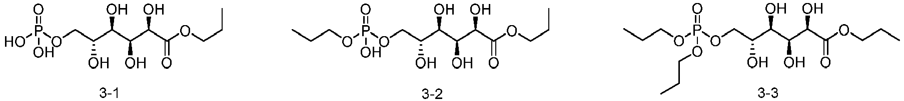

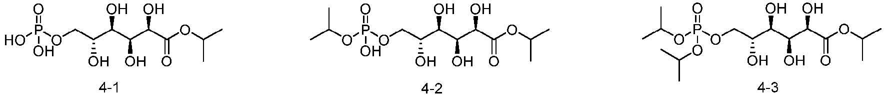

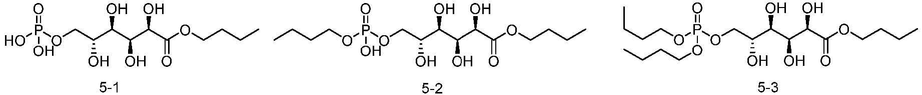

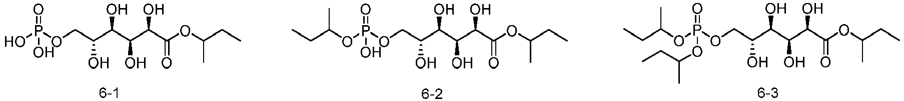

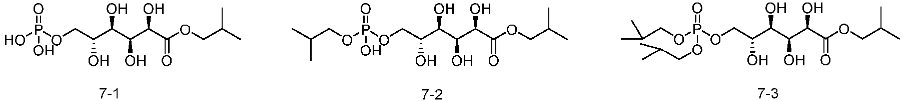

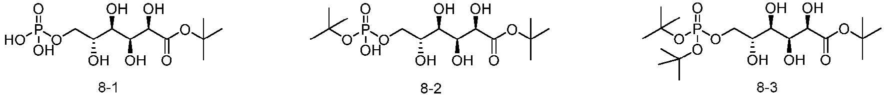

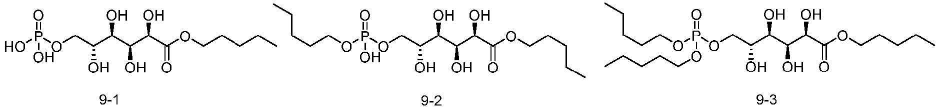

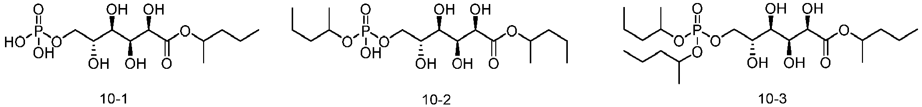

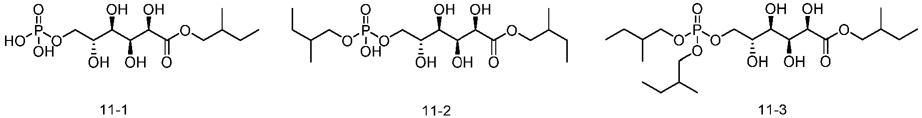

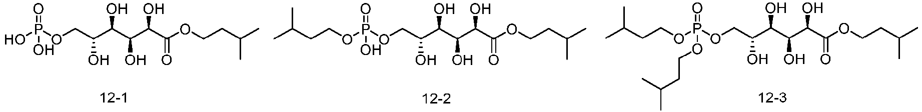

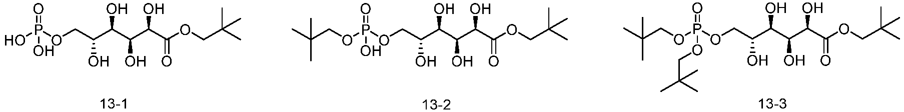

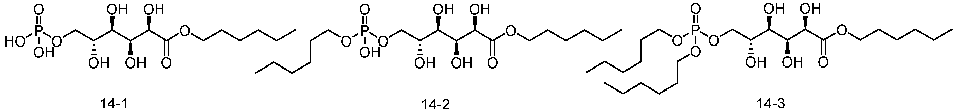

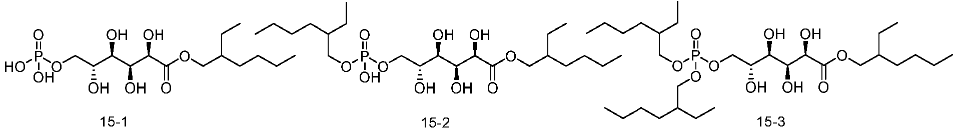

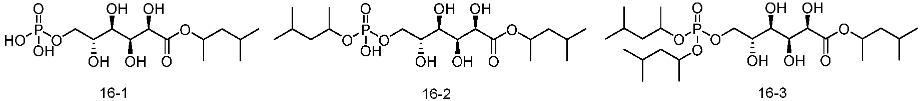

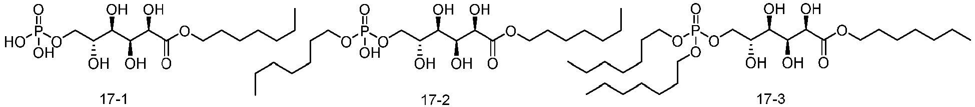

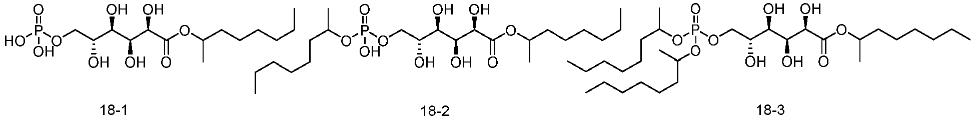

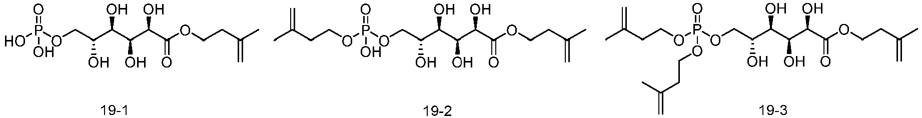

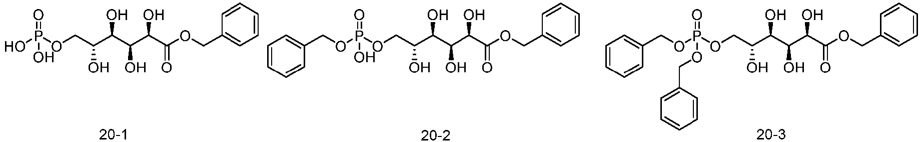

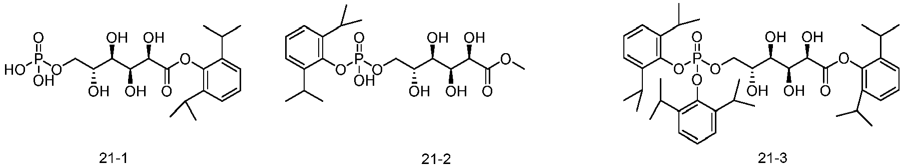

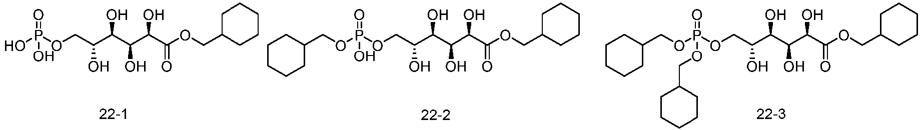

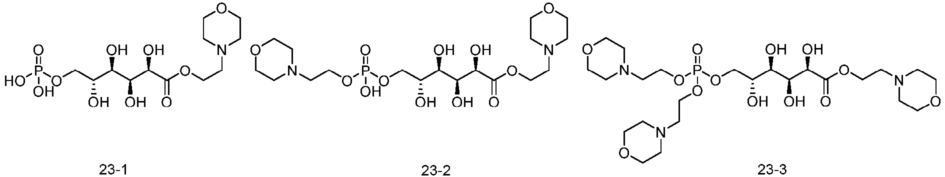

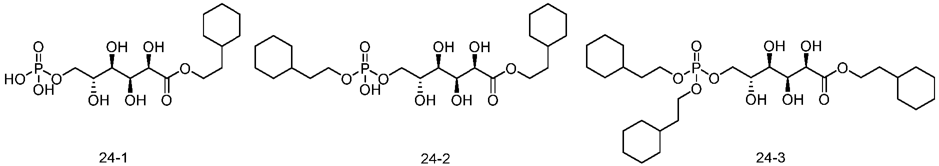

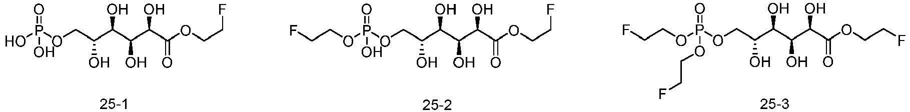

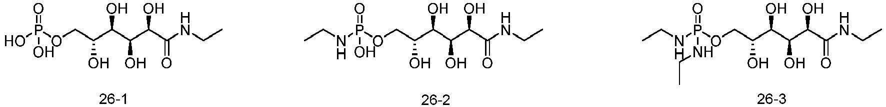

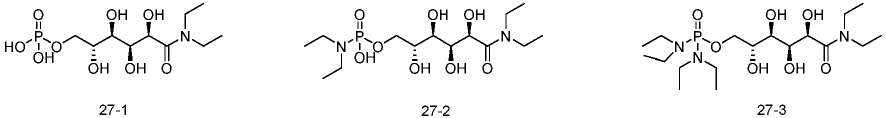

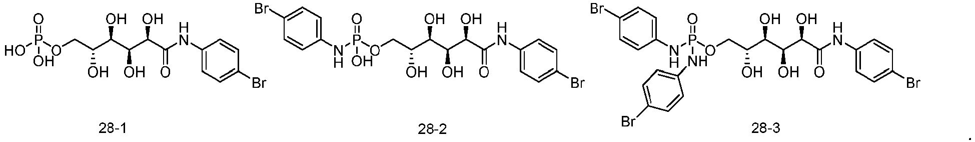

The scope of the present disclosure is not intended to be exhaustive of synthesis of 6-phosphogluconic acid as a prodrug for all the carboxylic ester derivatives, phosphate ester derivatives, amide derivatives, phosphoramide derivatives, thiophosphate ester derivatives, aminocarboxylic ester derivatives, thioester of carboxylic acid derivatives, hydroxy derivatives, hydroxy ester derivatives, hydroxy amide derivative, thiol derivatives, substituted thiol derivatives, thiol ester derivatives, phosphonate derivatives, mono-/polyphosphonate ester derivatives, phosphine derivatives, phosphorane ylide derivatives, phosphate derivatives, aminophosphate ester derivatives, hypophosphite derivatives, phosphine oxide derivatives, thiophosphate derivatives, fluorophosphate derivatives, phosphoric anhydride derivatives, bisphosphonate derivatives, phosphite ester derivatives, phosphonite derivatives, dithiophosphate ester derivatives, phosphonamide derivatives, and halogenated derivatives. It is certain that each derivative of 6-phosphogluconic acid can be regarded as a derivative formed by modifying and connecting 6-phosphogluconic acid with combinations of the fragments selected from carboxylic ester, phosphate ester, amid, phosphoramide, thiophosphate ester, aminocarboxylic ester, thioester of carboxylic acid, hydroxy, hydroxy ester, hydroxy amide, thiol, substituted thiol, thiol ester, phosphonate, mono-/polyphosphonate ester, phosphine, phosphorane ylide, phosphate, aminophosphate ester, hypophosphite, phosphine oxide, thiophosphate, fluorophosphate, phosphoric anhydride, bisphosphonate, phosphite ester, phosphonite, dithiophosphate ester, phosphonamide, and halogenated, in which each fragment can be modified by any possible modification strategies related to the functional groups of 6-phosphogluconic acid in existing art.

The term "pharmaceutically acceptable salts" in the present disclosure includes sodium salts, potassium salts, calcium salts, ammonium salts, amino acid salts, and other salts suitable as pharmaceutically acceptable salts.

Some of the compounds in the present disclosure contain acidic groups that capable of forming salts with bases, and derivatives of such salts can be prepared using conventional methods, which including sodium salt, potassium salt, calcium salt, ammonium salt, or amino acid salt such as lysine, arginine and histidine, and a preferred salt is sodium salt and potassium salt. Similarly, some of the compounds in the present disclosure contain alkaline groups that capable of forming salts with acids, and derivatives of such salts can be prepared using conventional methods, which including organic salts such as acetate, citrate, fumarate, maleate, oxalate, malate, citrate, succinate, tartrate, lactate, camphor sulfonate, benzene sulfonate, p-toluenesulfonate, methanesulfonate, trifluoroacetate, and trifluoromethanesulfonic salts; and, inorganic salt such as hydrohalide salts (e.g. hydrofluoride, hydrochloride, hydrobromide or hydroiodide), sulfate, phosphate, and nitrate. The compounds in the present disclosure can also form salts with amino acid, such as glutamic acid or aspartic acid, to form glutamate or aspartate. A preferred salt is hydrochloride and hydrobromide.

The term "halogen/ halogens" refers to fluorine, chlorine, bromine and iodine, preferably fluorine and chlorine.

The term "alkyl" refers to a saturated aliphatic group, including straight chain alkyl group such as methyl, ethyl, propyl, butyl, pentyl, hexyl, heptyl, octyl, nonyl, decyl, etc.; branched chain alkyl group such as isopropyl, tert-butyl, isobutyl, etc.; cycloalkyl group such as cyclopropyl, cyclopentyl, cyclohexyl, cycloheptyl and cyclooctyl; alkylsubstituted cycloalkyl group, cycloalkyl-substituted alkyl group, and alkyl group or cycloalkyl group substituted with other functional groups. In certain embodiments, the straight or branched chain alkyl group contains 4 or fewer carbon atoms in the skeletal.

The term "alkenyl" refers to an unsaturated aliphatic group containing one or more carbon-carbon double bonds, including straight chain alkenyl group such as vinyl, propenyl, butenyl, pentenyl, hexenyl, heptenyl, octenyl, nonenyl, decenyl, etc.; branched chain alkenyl group such as isopropenyl, isobutenyl, etc.; cycloalkenyl group such as cyclopropenyl, cyclobutenyl, cyclopentenyl, cyclohexenyl, cycloheptenyl, cyclooctenyl, etc.; alkenyl-substituted cycloalkenyl group, cycloalkenyl-substituted alkenyl group, and alkenyl group or cycloalkenyl group substituted with other functional groups. In certain embodiments, the straight or branched chain alkenyl group contains 4 or fewer carbon atoms in the skeletal.

The term "alkynyl" refers to an unsaturated aliphatic group containing one or more carbon-carbon triple bonds, including straight chain alkynyl group such as ethynyl, propynyl, butynyl, pentynyl, hexynyl, heptynyl, octynyl, nonenyl, decynyl, etc.; branched alkynyl group such as isopentynyl, isohexynyl, etc.; and alkynyl group substituted with other functional groups. In certain embodiments, the straight or branched chain alkynyl group contains 4 or fewer carbon atoms in the skeletal.

The pharmaceutically acceptable drug delivery vehicle in the present disclosure may assist 6-phosphogluconic acid and derivatives thereof to penetrate the cell membrane and be absorbed by islet α-cells by combining with or encapsulating 6-phosphogluconic acid and derivatives thereof. The drug delivery vehicle is a nano drug delivery system of drug compound/composition solutions or suspensions, which includes liposomes, polymeric micelles or microcapsules, lipid nanoparticles, and chitosan nanoparticles. The 6-phosphogluconic acid or a derivative or a pharmaceutically acceptable salt thereof is encapsulated in the compound or nano delivery system.

The pharmaceutical composition of the present disclosure may further comprise one or more pharmaceutically acceptable carriers other than the pharmaceutically acceptable drug delivery vehicle. The carrier includes conventional diluents, excipients, fillers, adhesives, wetting agents, disintegrants, absorption promoters, surfactants, adsorption carriers, lubricants, etc. in the pharmaceutical field, and may also be added with a flavoring agent, a sweetening agent, etc. if necessary.

The pharmaceutical compositions of the present disclosure may be prepared in any form, such as granules, powders, tablets, coated tablets, capsules, pills, syrups, drops, solutions, suspensions and emulsions, or sustained-release formulations of an active ingredients, wherein the capsules include hard or soft gelatin capsules, the granules and the powders may be in non-effervescent or effervescent forms.

The pharmaceutical compositions of the present disclosure can be administered by various routes according to conventional methods including oral administration, intravenous administration, intraarterial administration, intraperitoneal administration, intrapleural administration, transdermal administration, nasal administration, inhalation administration, rectal administration, ophthalmic drug delivery and subcutaneous introduction.

The effect of 6-phosphogluconic acid and the derivatives thereof including the pharmaceutically acceptable salts as a metabolism regulator on the increase of the transcription level of insulin synthesis-related genes in islet α-cells in vitro is determined by using quantitative real-time polymerase chain reaction (q-PCR) reported in literatures. q-PCR mainly monitors the changes in the amounts of amplified products in each cycle of the PCR amplification reaction in real time by means of changing in fluorescence signals, in which commonly used fluorescent labeling methods include SYBR green I dye and TaqMan probe. Finally, the effect of metabolism regulator on the increase of the transcription level of insulin synthesis-related genes in islet α-cells is determined by quantitatively analyzing the starting template by using the relationship between Ct values and standard curves.

It has been verified by experiments that the compounds disclosed in the present disclosure are a type of novel cell glucose metabolism regulators and diabetes treatment agents, which have a significant regulation effect on islet α-cells and extremely low toxicity due to their derivation from endogenous metabolites. The compounds can be further prepared into a medicament for preventing or treating diabetes and other glucose metabolism disorder diseases including diabetes related complications, such as diabetic ketoacidosis, hyperosmolar hyperglycemic nonketotic coma, diabetic retinopathy, diabetic nephropathy and diabetic foot; cardiovascular and cerebrovascular diseases, such as microangiopathy on heart or major vessels, cardiomyopathy, coronary heart disease, cerebral arteriosclerosis, ischemic cerebrovascular disease, etc.; and central nervous system diseases, such as neurodegenerative diseases including Alzheimer's disease and Parkinson's disease.

The advantages of the present disclosure are that the 6-phosphogluconic acid and the derivatives thereof, including the carboxylates/phosphates derived therefrom as prodrugs, are a type of novel cell glucose metabolism regulators and diabetes treatment agents, which have a particular significant regulation effect on islet α-cells and can be further prepared into a medicament for preventing or treating diabetes and other glucose metabolism disorder diseases.

### BRIEF DESCRIPTION OF THE DRAWINGS

Fig. 1 illustrates the differences in metabolites between islet α-cells and islet β-cells;
Fig. 2 illustrates the LC-MS/MS experiment targeted detection of 6-Phosphogluconic Acid;
Fig. 3 illustrates the significantly increases in transcription level of insulin synthesis-related genes in islet α-cells treated with target compound at a concentration of 10 µM;
Fig. 4 illustrates the verification of the regenerative effect of 6-phosphogluconic acid methyl ester on islet beta cells;
Fig. 5 illustrates the regeneration of islet beta cells of type 1 diabetes mellitus mice measured by an immunofluorescence assay;
Fig. 6 illustrates the concentration of insulin in peripheral blood of mice measured by an ultrasensitive insulin kit.

### DETAILED DESCRIPTION

The present invention will be described in detail below with reference to specific embodiments. One skilled in the relevant art will understand that the embodiments disclosed hereinafter are to provide a thorough understanding of the present disclosure, rather than limiting the present disclosure.

### Example 1. Determination and comparison of the metabolite concentration of islet α-cells and islet β-cells

### 1) Metabolomics analysis of islet α-cells and islet β-cells

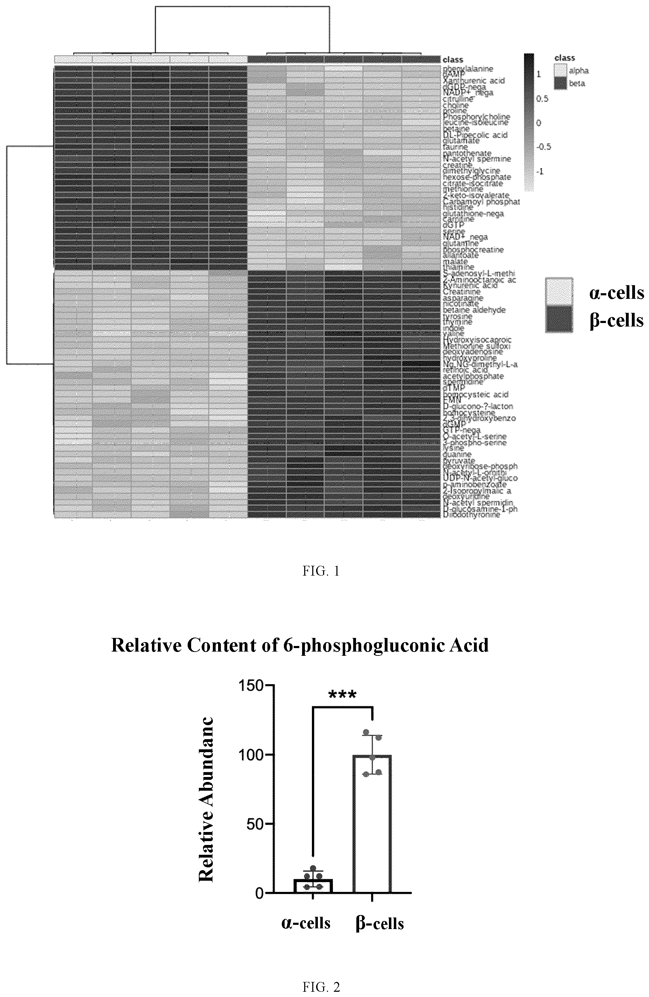

1×10⁶islet α-cells and 1×10⁶islet beta cells were independently placed in a 15 mL centrifuge tube, followed by adding 4.5 mL of the mixed solution of methanol and water, in which the volume ratio of methanol to water was 4:1 and having a temperature of -80°C, and incubated at a temperature of -80°C for 20 minutes. The centrifuge tube was then taken out, oscillated and vortexed for 1 minute, followed by being centrifuged at 14,000 × g for 5 minutes. Supernatants were then taken out and placed in a new centrifuge tube, followed by being dried by means of vacuum centrifugal drying or nitrogen blowing instrument. The dried metabolites were then redissolved with 100 µl of 35% acetonitrile solution. The metabolome in islet α-cells and islet β-cells were determined through LC-MS / MS based targeted metabolomics method. As illustrated in Fig.1, the heat map of the results of metabolomics analysis indicated that there were significant differences between islet α-cells and islet β-cells.

### 2) Detection of 6-phosphogluconic acid in islet α-cells and islet β-cells

6-phosphogluconic acid in islet α-cells and islet β-cells were independently extracted according to the above-mentioned metabolite extraction method. The dried metabolites were then redissolved with 100 µl of 35% acetonitrile solution. The 6-phosphogluconic acid in islet α-cells and islet β-cells were determined through LC-MS/MS based targeted metabolomics method, wherein MRM: *m*/*z* 275 -> 79, DP=-93V, and CE=-10V. As illustrated in Fig. 2, the results of LC-MS/MS experiment indicated that the concentration of 6-phosphogluconic acid in islet β-cells was relatively high.

### Example 2. Preparation of 6-phosphogluconic acid methyl ester, a derivative of 6-phosphogluconic acid of Formula 1-1

The compound of Formula (I) was obtained from 6-phosphogluconic acid esterified with a corresponding alcohol under acid catalysis. The reaction conditions were generally as follows: dry alcohol was used as a solvent, and dry HCl gas was introduced as a catalyst. The 6-phosphogluconic acid is a known compound and can be obtained by phosphorylation and oxidation of glucose. The following reaction equation and synthesis conditions set forth the synthesis method of Formula (1) in detail.
1) Synthesis of Glucose-6-Phosphate 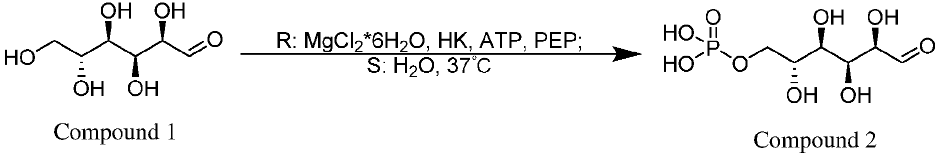
2) Synthesis of 6-phosphogluconic acid 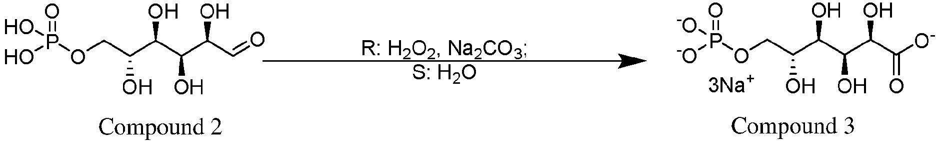
3) Synthesis of 6-phosphogluconic acid methyl ester of Compound 1-1, General Synthetic Method 1

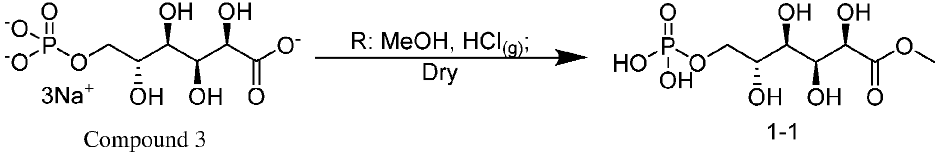

1 g (0.0029 mol, 1.0 eq) of compound 3 was weighed and placed into a 50 mL three-necked flask, followed by adding 25 mL of anhydrous methanol for dissolution. Dry hydrogen chloride gas was introduced into the reaction solution while stirring, and then stirred overnight and detected the reaction by using LC-MS. An oily crude product was obtained by being concentrated after vacuum distillation. The oily crude product was then dissolved by HPLC grade methanol and purified by HPLC to obtain 0.7 g of white solid, that is, 6-phosphogluconic acid methyl ester of Formula 1-1, with a yield of 82%. MS(ESI) (*m*/*z*)*:* 289[M-H]⁻. ¹H NMR (400 MHz, DMSO-*d6*)δ 5.18 (s, 1H), 4.51 (s, 1H), 4.37 (s, 2H), 4.33 (m, 1H), 4.29 (m, 2H), 4.2 (s, 2H), 4.13 (d, 1H), 3.70 (s, 3H), 3.50 (m, 1H), 3.40 (m, 1H).

### Example 3. Preparation of Compounds 1-2, 1-3, 2-1, 2-2, 2-3, 3-1, 3-2, 3-3, 4-1, 4-2, 4-3, 5-1, 5-2, 5-3, 6-1, 6-2, 6-3, 7-1, 7-2, 7-3, 8-1, 8-2, 8-3, 9-1, 9-2, 9-3, 10-1, 10-2, 10-3, 11-1, 11-2, 11-3, 12-1, 12-2, 12-3, 13-1, 13-2, 13-3, 14-1, 14-2, 14-3, 15-1, 15-2, 15-3, 16-1, 16-2, 16-3, 17-1, 17-2, 17-3, 18-1, 18-2, 18-3, 19-1, 19-2, 19-3, 20-1, 20-2, 20-3, 21-1, 21-2, 21-3, 22-1, 22-2, 22-3, 23-1, 23-2, 23-3, 24-1, 24-2, 24-3, 25-1, 25-2, 25-3

Referring to the conditions and steps of the General Synthetic Method 1 in Example 2, 6- phosphogluconic acid dimethyl ester of Formula 1-2 was prepared by reacting anhydrous methanol with compound 3, with a yield of 20%.

Referring to the conditions and steps of the General Synthetic Method 1 in Example 2, 6- phosphogluconic acid trimethyl ester of Formula 1-3 was prepared by reacting anhydrous methanol with compound 3, with a yield of 10%.

Referring to the conditions and steps of the General Synthetic Method 1 in Example 2, 6- phosphogluconic acid ethyl ester of Formula 2-1 was prepared by reacting anhydrous ethanol with compound 3, with a yield of 70%.

Referring to the conditions and steps of the General Synthetic Method 1 in Example 2, 6- phosphogluconic acid diethyl ester of Formula 2-2 was prepared by reacting anhydrous ethanol with compound 3, with a yield of 20%.

Referring to the conditions and steps of the General Synthetic Method 1 in Example 2, 6- phosphogluconic acid triethyl ester of Formula 2-3 was prepared by reacting anhydrous ethanol with compound 3, with a yield of 10%.

Referring to the conditions and steps of the General Synthetic Method 1 in Example 2, 6- phosphogluconic acid propyl ester of Formula 3-1 was prepared by reacting anhydrous propanol with compound 3, with a yield of 70%.

Referring to the conditions and steps of the General Synthetic Method 1 in Example 2, 6- phosphogluconic acid dipropyl ester of Formula 3-2 was prepared by reacting anhydrous propanol with compound 3, with a yield of 20%.

Referring to the conditions and steps of the General Synthetic Method 1 in Example 2, 6- phosphogluconic acid tripropyl ester of Formula 3-3 was prepared by reacting anhydrous propanol with compound 3, with a yield of 10%.

Referring to the conditions and steps of the General Synthetic Method 1 in Example 2, 6- phosphogluconic acid isopropanol ester of Formula 4-1 was prepared by reacting anhydrous isopropyl alcohol with compound 3, with a yield of 70%.

Referring to the conditions and steps of the General Synthetic Method 1 in Example 2, 6- phosphogluconic acid di-isopropanol ester of Formula 4-2 was prepared by reacting anhydrous isopropyl alcohol with compound 3, with a yield of 20%.

Referring to the conditions and steps of the General Synthetic Method 1 in Example 2, 6- phosphogluconic acid tri-isopropanol ester of Formula 4-3 was prepared by reacting anhydrous isopropyl alcohol with compound 3, with a yield of 10%.

Referring to the conditions and steps of the General Synthetic Method 1 in Example 2, 6- phosphogluconic acid butyl ester of Formula 5-1 was prepared by reacting anhydrous butyl alcohol with compound 3, with a yield of 70%.

Referring to the conditions and steps of the General Synthetic Method 1 in Example 2, 6- phosphogluconic acid dibutyl ester of Formula 5-2 was prepared by reacting anhydrous butyl alcohol with compound 3, with a yield of 20%.

Referring to the conditions and steps of the General Synthetic Method 1 in Example 2, 6- phosphogluconic acid tri-n-butyl ester of Formula 5-3 was prepared by reacting anhydrous butyl alcohol with compound 3, with a yield of 10%.

Referring to the conditions and steps of the General Synthetic Method 1 in Example 2, 6- phosphogluconic acid sec-butyl ester of Formula 6-1 was prepared by reacting anhydrous 2-nutanol with compound 3, with a yield of 70%.

Referring to the conditions and steps of the General Synthetic Method 1 in Example 2, 6- phosphogluconic acid di-sec-butyl ester of Formula 6-2 was prepared by reacting anhydrous 2-nutanol with compound 3, with a yield of 20%.

Referring to the conditions and steps of the General Synthetic Method 1 in Example 2, 6- phosphogluconic acid tri-sec-butyl ester of Formula 6-3 was prepared by reacting anhydrous 2-nutanol with compound 3, with a yield of 10%.

Referring to the conditions and steps of the General Synthetic Method 1 in Example 2, 6- phosphogluconic acid isobutyl ester of Formula 7-1 was prepared by reacting anhydrous isobutyl alcohol with compound 3, with a yield of 70%.

Referring to the conditions and steps of the General Synthetic Method 1 in Example 2, 6- phosphogluconic acid di-isobutyl ester of Formula 7-2 was prepared by reacting anhydrous isobutyl alcohol with compound 3, with a yield of 20%.

Referring to the conditions and steps of the General Synthetic Method 1 in Example 2, 6- phosphogluconic acid tri-isobutyl ester of Formula 7-3 was prepared by reacting anhydrous isobutyl alcohol with compound 3, with a yield of 10%.

Referring to the conditions and steps of the General Synthetic Method 1 in Example 2, 6- phosphogluconic acid tert-butyl ester of Formula 8-1 was prepared by reacting anhydrous tert-butyl alcohol with compound 3, with a yield of 70%.

Referring to the conditions and steps of the General Synthetic Method 1 in Example 2, 6- phosphogluconic acid di-tert-butyl ester of Formula 8-2 was prepared by reacting anhydrous tert-butyl alcohol with compound 3, with a yield of 20%.

Referring to the conditions and steps of the General Synthetic Method 1 in Example 2, 6- phosphogluconic acid tri-tert-butyl ester of Formula 8-3 was prepared by reacting anhydrous tert-butyl alcohol with compound 3, with a yield of 10%.

Referring to the conditions and steps of the General Synthetic Method 1 in Example 2, 6- phosphogluconic acid pentyl ester of Formula 9-1 was prepared by reacting anhydrous pentanol with compound 3, with a yield of 70%.

Referring to the conditions and steps of the General Synthetic Method 1 in Example 2, 6- phosphogluconic acid di-pentyl ester of Formula 9-2 was prepared by reacting anhydrous pentanol with compound 3, with a yield of 20%.

Referring to the conditions and steps of the General Synthetic Method 1 in Example 2, 6- phosphogluconic acid tri-pentyl amyl ester of Formula 9-3 was prepared by reacting anhydrous pentanol with compound 3, with a yield of 10%.

Referring to the conditions and steps of the General Synthetic Method 1 in Example 2, 6- phosphogluconic acid sec-pentanol ester of Formula 10-1 was prepared by reacting anhydrous 2-pentanol with compound 3, with a yield of 70%.

Referring to the conditions and steps of the General Synthetic Method 1 in Example 2, 6- phosphogluconic acid di-sec-pentanol ester of Formula 10-2 was prepared by reacting anhydrous 2-pentanol with compound 3, with a yield of 20%.

Referring to the conditions and steps of the General Synthetic Method 1 in Example 2, 6- phosphogluconic acid tri-sec-pentanol ester of Formula 10-3 was prepared by reacting anhydrous 2-pentanol with compound 3, with a yield of 10%.

Referring to the conditions and steps of the General Synthetic Method 1 in Example 2, 6- phosphogluconic acid 2-methyl butanol ester of Formula 11-1 was prepared by reacting anhydrous 2-methyl-1-butanol with compound 3, with a yield of 70%.

Referring to the conditions and steps of the General Synthetic Method 1 in Example 2, 6- phosphogluconic acid 2-methyl di-butanol ester of Formula 11-2 was prepared by reacting anhydrous 2-methyl-1-butanol with compound 3, with a yield of 20%.

Referring to the conditions and steps of the General Synthetic Method 1 in Example 2, 6- phosphogluconic acid 2-methyl tri-butanol ester of Formula 11-3 was prepared by reacting anhydrous 2-methyl-1-butanol with compound 3, with a yield of 10%.

Referring to the conditions and steps of the General Synthetic Method 1 in Example 2, 6- phosphogluconic acid isopentyl ester of Formula 12-1 was prepared by reacting anhydrous isopentanol with compound 3, with a yield of 70%.

Referring to the conditions and steps of the General Synthetic Method 1 in Example 2, 6- phosphogluconic acid di-isopentyl ester of Formula 12-2 was prepared by reacting anhydrous isopentanol with compound 3, with a yield of 20%.

Referring to the conditions and steps of the General Synthetic Method 1 in Example 2, 6- phosphogluconic acid tri-isopentyl ester of Formula 12-3 was prepared by reacting anhydrous isopentanol with compound 3, with a yield of 10%.

Referring to the conditions and steps of the General Synthetic Method 1 in Example 2, 6- phosphogluconic acid neopentyl ester of Formula 13-1 was prepared by reacting anhydrous neopentanol with compound 3, with a yield of 70%.

Referring to the conditions and steps of the General Synthetic Method 1 in Example 2, 6- phosphogluconic acid di-neopentyl ester of Formula 13-2 was prepared by reacting anhydrous neopentanol with compound 3, with a yield of 20%.

Referring to the conditions and steps of the General Synthetic Method 1 in Example 2, 6- phosphogluconic acid tri-neopentyl ester of Formula 13-3 was prepared by reacting anhydrous neopentanol with compound 3, with a yield of 10%.

Referring to the conditions and steps of the General Synthetic Method 1 in Example 2, 6- phosphogluconic acid hexyl ester of Formula 14-1 was prepared by reacting anhydrous 1-hexanol with compound 3, with a yield of 70%.

Referring to the conditions and steps of the General Synthetic Method 1 in Example 2, 6- phosphogluconic acid di-hexyl ester of Formula 14-2 was prepared by reacting anhydrous 1-hexanol with compound 3, with a yield of 20%.

Referring to the conditions and steps of the General Synthetic Method 1 in Example 2, 6- phosphogluconic acid tri-hexyl ester of Formula 14-3 was prepared by reacting anhydrous 1-hexanol with compound 3, with a yield of 10%.

Referring to the conditions and steps of the General Synthetic Method 1 in Example 2, 6- phosphogluconic acid 2-ethylhexyl ester of Formula 15-1 was prepared by reacting anhydrous 2-ethylhexanol with compound 3, with a yield of 70%.

Referring to the conditions and steps of the General Synthetic Method 1 in Example 2, 6- phosphogluconic acid di-2-ethylhexyl ester of Formula 15-2 was prepared by reacting anhydrous 2-ethylhexanol with compound 3, with a yield of 20%.

Referring to the conditions and steps of the General Synthetic Method 1 in Example 2, 6- phosphogluconic acid tri-2-ethylhexyl ester of Formula 15-3 was prepared by reacting anhydrous 2-ethylhexanol with compound 3, with a yield of 10%.

Referring to the conditions and steps of the General Synthetic Method 1 in Example 2, 6- phosphogluconic acid 1,3-dimethylbutyl ester of Formula 16-1 was prepared by reacting anhydrous 1,3-dimethyl-1-butanol with compound 3, with a yield of 70%.

Referring to the conditions and steps of the General Synthetic Method 1 in Example 2, 6- phosphogluconic acid di-1,3-dimethylbutyl ester of Formula 16-2 was prepared by reacting anhydrous 1,3-dimethyl-1-butanol with compound 3, with a yield of 20%.

Referring to the conditions and steps of the General Synthetic Method 1 in Example 2, 6- phosphogluconic acid tri-1,3-dimethylbutyl ester of Formula 16-3 was prepared by reacting anhydrous 1,3-dimethyl-1-butanol with compound 3, with a yield of 10%.

Referring to the conditions and steps of the General Synthetic Method 1 in Example 2, 6- phosphogluconic acid heptyl ester of Formula 17-1 was prepared by reacting anhydrous 1-heptanol with compound 3, with a yield of 70%.

Referring to the conditions and steps of the General Synthetic Method 1 in Example 2, 6- phosphogluconic acid di-heptyl ester of Formula 17-2 was prepared by reacting anhydrous 1-heptanol with compound 3, with a yield of 20%.

Referring to the conditions and steps of the General Synthetic Method 1 in Example 2, 6- phosphogluconic acid tri-heptyl ester of Formula 17-3 was prepared by reacting anhydrous 1-heptanol with compound 3, with a yield of 10%.

Referring to the conditions and steps of the General Synthetic Method 1 in Example 2, 6- phosphogluconic acid methyl heptyl ester of Formula 18-1 was prepared by reacting anhydrous 1-methylheptanol with compound 3, with a yield of 70%.

Referring to the conditions and steps of the General Synthetic Method 1 in Example 2, 6- phosphogluconic acid methyl di-heptyl ester of Formula 18-2 was prepared by reacting anhydrous 1-methylheptanol with compound 3, with a yield of 20%.

Referring to the conditions and steps of the General Synthetic Method 1 in Example 2, 6- phosphogluconic acid methyl tri-heptyl ester of Formula 18-3 was prepared by reacting anhydrous 1-methylheptanol with compound 3, with a yield of 10%.

Referring to the conditions and steps of the General Synthetic Method 1 in Example 2, 6- phosphogluconic acid 3-methyl-3-buten-1-ol ester of Formula 19-1 was prepared by reacting anhydrous 3-methyl-3-buten-1-ol with compound 3, with a yield of 70%.

Referring to the conditions and steps of the General Synthetic Method 1 in Example 2, 6- phosphogluconic acid 3-methyl-3-buten-1-ol di-ester of Formula 19-2 was prepared by reacting anhydrous 3-methyl-3-buten-1-ol with compound 3, with a yield of 20%.

Referring to the conditions and steps of the General Synthetic Method 1 in Example 2, 6- phosphogluconic acid 3-methyl-3-buten-1-ol tri-ester of Formula 19-3 was prepared by reacting anhydrous 3-methyl-3-buten-1-ol with compound 3, with a yield of 10%.

Referring to the conditions and steps of the General Synthetic Method 1 in Example 2, 6- phosphogluconic acid methyl phenyl ester of Formula 20-1 was prepared by reacting anhydrous benzyl alcohol with compound 3, with a yield of 70%.

Referring to the conditions and steps of the General Synthetic Method 1 in Example 2, 6- phosphogluconic acid methyl phenyl di-ester of Formula 20-2 was prepared by reacting anhydrous benzyl alcohol with compound 3, with a yield of 20%.

Referring to the conditions and steps of the General Synthetic Method 1 in Example 2, 6- phosphogluconic acid methyl phenyl tri-ester of Formula 20-3 was prepared by reacting anhydrous benzyl alcohol with compound 3, with a yield of 10%.

Referring to the conditions and steps of the General Synthetic Method 1 in Example 2, 6- phosphogluconic acid 2,6-diisopropylphenyl ester of Formula 21-1 was prepared by reacting anhydrous 2,6-diisopropylphenol with compound 3, with a yield of 70%.

Referring to the conditions and steps of the General Synthetic Method 1 in Example 2, 6- phosphogluconic acid 2,6-diisopropylphenyl di-ester of Formula 21-2 was prepared by reacting anhydrous 2,6-diisopropylphenol with compound 3, with a yield of 20%.

Referring to the conditions and steps of the General Synthetic Method 1 in Example 2, 6- phosphogluconic acid 2,6-diisopropylphenyl tri-ester of Formula 21-3 was prepared by reacting anhydrous 2,6-diisopropylphenol with compound 3, with a yield of 10%.

Referring to the conditions and steps of the General Synthetic Method 1 in Example 2, 6- phosphogluconic acid cyclohexyl methyl ester of Formula 22-1 was prepared by reacting anhydrous cyclohexanemethanol with compound 3, with a yield of 70%.

Referring to the conditions and steps of the General Synthetic Method 1 in Example 2, 6- phosphogluconic acid cyclohexyl methyl di-ester of Formula 22-2 was prepared by reacting anhydrous cyclohexanemethanol with compound 3, with a yield of 20%.

Referring to the conditions and steps of the General Synthetic Method 1 in Example 2, 6- phosphogluconic acid cyclohexyl methyl tri-ester of Formula 22-3 was prepared by reacting anhydrous cyclohexanemethanol with compound 3, with a yield of 10%.

Referring to the conditions and steps of the General Synthetic Method 1 in Example 2, 6- phosphogluconic acid 2-morpholinoethyl ester of Formula 23-1 was prepared by reacting anhydrous 2-morpholinoethanol with compound 3, with a yield of 70%.

Referring to the conditions and steps of the General Synthetic Method 1 in Example 2, 6- phosphogluconic acid 2-morpholine diethyl ester of Formula 23-2 was prepared by reacting anhydrous 2-morpholinoethanol with compound 3, with a yield of 20%.

Referring to the conditions and steps of the General Synthetic Method 1 in Example 2, 6- phosphogluconic acid 2-morpholine triethyl ester of Formula 23-3 was prepared by reacting anhydrous 2-morpholinoethanol with compound 3, with a yield of 10%.

Referring to the conditions and steps of the General Synthetic Method 1 in Example 2, 6- phosphogluconic acid cyclohexyl ethyl ester of Formula 24-1 was prepared by reacting anhydrous cyclohexylethanol with compound 3, with a yield of 70%.

Referring to the conditions and steps of the General Synthetic Method 1 in Example 2, 6- phosphogluconic acid cyclohexyl diethyl ester of Formula 24-2 was prepared by reacting anhydrous cyclohexylethanol with compound 3, with a yield of 20%.

Referring to the conditions and steps of the General Synthetic Method 1 in Example 2, 6- phosphogluconic acid cyclohexyl triethyl ester of Formula 24-3 was prepared by reacting anhydrous cyclohexylethanol with compound 3, with a yield of 10%.

Referring to the conditions and steps of the General Synthetic Method 1 in Example 2, 6- phosphogluconic acid 2-fluoroethyl ester of Formula 25-1 was prepared by reacting anhydrous 2-fluoroethanol with compound 3, with a yield of 70%.

Referring to the conditions and steps of the General Synthetic Method 1 in Example 2, 6- phosphogluconic acid 2-fluorodiethyl ester of Formula 25-2 was prepared by reacting anhydrous 2-fluoroethanol with compound 3, with a yield of 20%.

Referring to the conditions and steps of the General Synthetic Method 1 in Example 2, 6- phosphogluconic acid 2-fluorotriethyl ester of Formula 25-3 was prepared by reacting anhydrous 2-fluoroethanol with compound 3, with a yield of 10%.

### Example 4. Preparation of N-ethyl-6-phosphoglucamide of Formula 26-1

### 1) Synthesis of N-ethyl-6-phosphoglucamide of Formula 26-1, General Synthesis Method 2

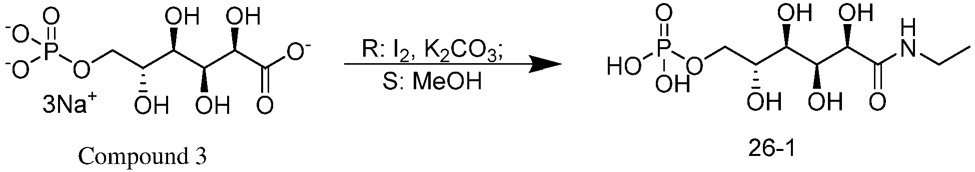

1 g (0.0029 mol, 1.0 eq) of Compound 3 was weighed and fully dissolved in 10 mL of methanol in a 50 ml round-bottomed flask. 0.47 g of ethylamine hydrochloride (0.006 mol, 2.0 eq), 1.47 g of iodine (0.006 mol, 2.0 eq) and 0.83 g of K₂CO₃ (0.006 mol, 2.0 eq) were weighed and added into methanol solution of Compound 3 while stirring. The reaction solution was stirred at room temperature for 12 hours and detected in real time by using LC-MS until the compound 3 was completely consumed. A yellow crude product was obtained by filtering the reaction solution and concentrating after vacuum distillation, followed by separated and purified by using a resin column (Dowex 8 WX-100, in acid form) to obtain 0.65 g of white solid, with a yield of 74%. MS(ESI)(m/z): 302[M-H]⁻. ^{l}H NMR (400 MHz, DMSO-d6) δ 8.01 (s, 1H), 5.18 (s, 1H), 4.51 (s, 1H), 4.44 (d, 1H) 4.37 (s, 2H), 4.29 (m, 2H), 4.20 (s, 2H), 3.80(m, 1H), 3.50 (m, 1H), 3.40 (m, 1H), 3.24(m, 2H), 0.99(m, 3H).

### Example 5. Preparation of Compounds 26-2, 26-3, 27-1, 27-2, 27-3, 28-1, 28-2, 28-3

Referring to the conditions and steps of the General Synthetic Method 2 in Example 4, di-N-ethyl-6-phosphoglucamide of Formula 26-2 was prepared by reacting anhydrous ethylamine hydrochloride with compound 3, with a yield of 20%.

Referring to the conditions and steps of the General Synthetic Method 2 in Example 4, tri-N-ethyl-6-phosphoglucamide of Formula 26-3 was prepared by reacting anhydrous ethylamine hydrochloride with compound 3, with a yield of 10%.

Referring to the conditions and steps of the General Synthetic Method 2 in Example 4, N,N-2-ethyl-6-phosphoglucamide of Formula 27-1 was prepared by reacting anhydrous diethylamine with compound 3, with a yield of 70%.

Referring to the conditions and steps of the General Synthetic Method 2 in Example 4, di-N,N-2-ethyl-6-phosphoglucamide of Formula 27-2 was prepared by reacting anhydrous diethylamine with compound 3, with a yield of 20%.

Referring to the conditions and steps of the General Synthetic Method 2 in Example 4, tri-N,N-2-ethyl-6-phosphoglucamide of Formula 27-3 was prepared by reacting anhydrous diethylamine with compound 3, with a yield of 10%.

Referring to the conditions and steps of the General Synthetic Method 2 in Example 4, N1-(4-bromophenyl)-6-phosphoglucamide of Formula 28-1 was prepared by reacting anhydrous 4-bromoaniline with compound 3, with a yield of 70%.

Referring to the conditions and steps of the General Synthetic Method 2 in Example 4, di-N1-(4-bromophenyl)-6-phosphoglucamide of Formula 28-2 was prepared by reacting anhydrous 4-bromoaniline with compound 3, with a yield of 20%.

Referring to the conditions and steps of the General Synthetic Method 2 in Example 4, tri-N1-(4-bromophenyl)-6-phosphoglucamide of Formula 28-3 was prepared by reacting anhydrous 4-bromoaniline with compound 3, with a yield of 10%.

### Example 6. Determination of the effect of 6-phosphogluconic acid and the derivatives thereof including the pharmaceutically acceptable salts on the increase of the transcription level of insulin synthesis-related genes in islet α-cells in vitro

### Experimental instruments and materials

1. q-PCR Test Kit, from Shanghai Beyotime Biotech Co., Ltd.;
2. QUANT STUDIO 3 Real-time Fluorescent Quantitative PCR System, from Thermo Fisher Scientific (China) Co., Ltd.;
4. Q-PCR Target Nucleic Acid Sequence Primer, from Beijing Tsingke Biotech Co., Ltd.;
3. Electric-heated thermostatic water bath, from Shanghai Yiheng Technology Co., Ltd.;
4. Vortex mixer, with model ID of XW-80 A, from Shanghai Jingke Industrial Co., Ltd.;
5. High speed centrifuge, with model ID of EPPENDORF 5804R;
6. Organic solvents purchased from Shanghai National Pharmaceutical Reagent Company, in which methanol was chromatographically pure, water was ultrapure water filtered by Milli-Q pump, deionized and ultrafiltrated by 0.22 µm membrane, and other biological consumables were purchased from domestic companies; and
7. A to-be-tested compound solution prepared: 1-2 mg of each compound to be tested was accurately weighed, followed by adding a proper amount of PBS to accurately prepare a 10 mmol/L stock solution. A certain volume of the PBS stock solution of the compound to be tested was taken, followed by adding an appropriate volume of PBS to dilute the compound to be tested to the solution with the required concentration.

The effect of 6-phosphogluconic acid and the derivatives thereof including the pharmaceutically acceptable salts on the increase of the transcription level of insulin synthesis-related genes in islet α-cells in vitro was determined as follows.

Samples were prepared as follows. Islet α-cells that had been administered over 72 hours were added Trizol and placed at room temperature for 5 minutes, so that the islet α-cells were fully cracked. After the lysis was completed, the lysate was centrifuged at 12,000 rmp at 4 °C for 5 min, and the supernatant was taken. The supernatant was added chloroform (200 ul/mL Trizol), followed by being placed at room temperature for 15 minutes after being gently shaken for 30 seconds, and then centrifuged at 12,000 rmp at 4 °C for 15 min to suck the supernatant into another centrifuge tube, in which isopropoxide with equal volume was then added. The mixture was gently shaken and uniformly mixed, standing for 15 min, and centrifuged at 12,000 rmp at 4 °C for 15 minutes, and then the precipitated RNA was left after discarding the supernatant. 1 ml of pre-cooled ethanol (75%), which was prepared with DEPC water when using, was added into the precipitated RNA. The centrifuge tube was gently shaken for 30 seconds, followed by being rinsed and precipitated. After centrifugation at 8,000 rmp at 4 °C for 5 minutes, all supernatant was discarded as much as possible. The sediment was dried at room temperature or dried in vacuum for 5-10 minutes. After ethanol was volatilized and the sediment became transparent, 40 µl of DEPC water was added to dissolve RNA, and then heated to 55-60 °C for dissolution.

OD values were measured to quantify RNA concentration as follows.

The sample obtained in the above-mentioned step was washed with DEPC water for three times, and the NanoDrop micro spectrophotometer was zeroed. 1 µl of the sample solution was taken for measurement to obtain the RNA concentration. The sample solution was then diluted to 3-4 ng/µl.

RNA reverse transcription was as follows.

DEPC water was added to a new test tube, and then 2 µg of RNA was added to configure a 12 µl of concentration test system. The concentration test system was then heated at 65 °C for 5 minutes, so that the RNA became a linear single chain, then rapidly placed on ice to stand for cooling for 2 minutes. Ncclease-free water and 4 µl (4 ×) of DNA master Mix, which had been added gDNA Remover, were added to the concentration test system to stand for reaction at 37 °C for 5 minutes, so that DNA of the genome was removed. 4 µl of q-PCR (5 ×) RT-Mix was added to configure a 20 µl of reaction system. The reaction system was then gently shaken and uniformly mixed. Then a reverse transcription reaction occurs according to the temperature and time sequence of 37 °C and 5 minutes → 50 °C and 5 minutes → 98 °C and 5 minutes to obtain a cDNA sample solution.

### q-PCR:

The q-PCR kit was prepared, pre-denaturation of the template was performed before the Real-time PCR reaction, which was set to 95 °C for 2 minutes, and then the q-PCR instrument was set according to the following parameters: pre-denaturation: 9 5°C, 2 minutes → denaturation: 95 °C, 15 seconds → annealing/extension: 60°C, 15-30 seconds → repeated the previous two steps, for a total of 40 cycles → melting curve analysis: 95 °C, 15 seconds; 60 °C, 15 seconds; 95 °C, 15 seconds. When the reaction was complete, the results were analyzed by using the software provided by the q-PCR instrument.

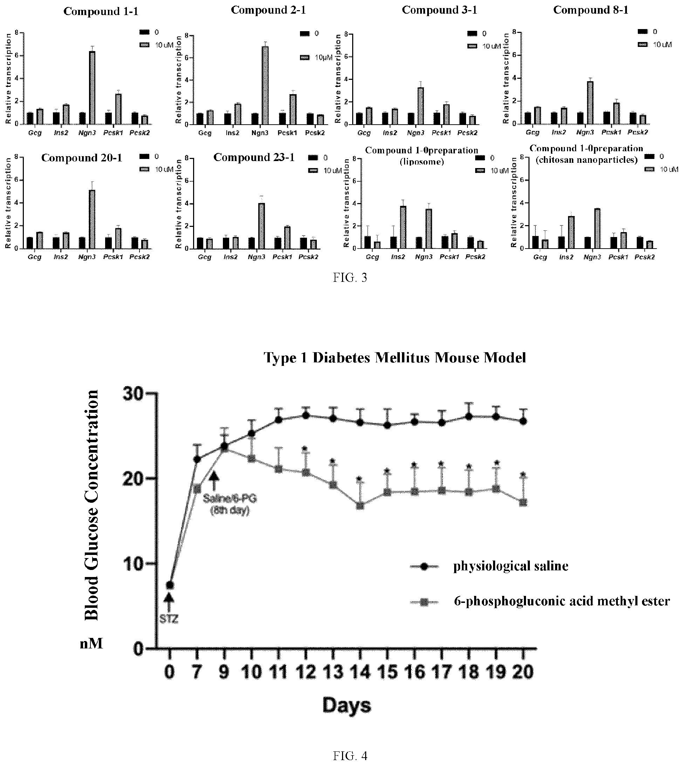

In the present embodiment, a representative dosage form of 6-phosphogluconic acid, such as liposome and chitosan nanoparticle, and 6 representative derivatives of 6-phosphogluconic acid were selected for testing. As illustrated in Fig. 3, the results indicated that the transcription level of insulin synthesis-related genes in islet α-cells treated with target compound at a concentration of 10 µM was significantly increased. The present embodiment is not intended to be exhaustive of all possible dosage forms and derivatives, for the reason that the 6-phosphogluconic acid can enter the cell to release 6-phosphogluconic acid as the effective component after being optimized or derivatized with the dosage form in theory, thereby achieving the same expected effect.

### Example 7. Preparation of chitosan nanoparticles of 6-phosphogluconic acid

### PRESCRIPTION

| | |
|---|---|
| Chitosan | 100 mg |
| Acetic acid | 0.2 mL |
| Tween 80 | 200 µL |
| 6-phosphogluconic acid | 50 mg |

Operation: first of all, the acetic acid (CH₃COOH, >99%) was diluted to an acetic acid solution with a volume fraction of 0.2%, and 200 µl of Tween-80 was dripped into the acetic acid solution. 100 mg of the chitosan was then dissolved in 100 ml acetic acid solution, stirred for 30 minutes until chitosan was completely dissolved. The solution was passed through a 0.22 µm filter membrane for later use. 5 ml of 6-phosphogluconic acid solution with a concentration of 10 mg/ml was taken and dripped into the chitosan solution at a rotation speed of 100 rpm to form nano microcapsules, which was then placed in a refrigerator at 4 °C for storage. When in experiment, the required amount of nanoparticle suspension was mixed with PBS of pH 6.8 and added to a cell culture dish for co incubation.

### Example 8. Preparation of liposomes of 6-phosphogluconic acid

### PRESCRIPTION

| | |
|---|---|
| DOPE | 75 mg |
| Chol | 38 mg |
| DOTAP | 75 mg |
| 6-phosphogluconic acid | 50 mg |

Operation: The dioleoyl phosphatidyl ethanolamine (DOPE), cholesterol (CHOL) and (1,2-diethoxypropyl) trimethyl ammonium chloride (DOTAP) were weighed according to the prescription and added into in a 250 ml round-mouth flask together with 15 ml of chloroform, and then be shaken until uniform and completely dissolved. The chloroform solvent was removed by vacuum distillation at 35 °C to form a transparent film, and the residual solvent was removed by vacuum drying at room temperature. 5-10 ml of 6-phosphogluconic acid solution with a concentration of 10 mg/ml was added, vortexed for 2 minutes first, and then hydrated at a constant temperature of 40 °C for 10 minutes to obtain a milky translucent liposome solution. The obtained liposome solution was placed in an ultrafiltration tube (100 Kraa MWCO), followed by being centrifuged at 4 °C and at 14,000 rpm for 15 minutes to obtain a concentrated liposome solution, which was then placed in a refrigerator at 4 °C for storage. When in experiment, the required amount of liposome solution was mixed with PBS of pH 6.8 and added to a cell culture dish for co incubation.

### Example 9. Preparation of liposomes of 6-phosphogluconic acid

### Preparation of liposomes of 6-phosphogluconic acid methyl ester

### PRESCRIPTION

| | |
|---|---|
| Soyabean lecithin | 11 mg |
| Chol | 9 mg |
| DSPE-PEG2000 | 1 mg |
| 6-phosphogluconic acid methyl ester | 50 mg |
| Chloroform | 10 ml |

Operation: The soyabean lecithin, Chol and DSPE-PEG 2000 were weighed according to the prescription and dissolved in 10mL of chloroform, and then a liposome film was formed by using rotary evaporation at 40 °C. 6-phosphogluconic acid methyl ester was dissolved in 10ml of PBS of pH 7.4, and then added into the liposome film for hydration. A cell pulverizer was then used to perform ultrasound, followed by membrane filtration. The obtained liposome solution was placed in an ultrafiltration tube (100 Kraa MWCO), followed by being centrifuged at 4 °C and at 14,000 rpm for 15 minutes to obtain a concentrated liposome solution, which was then placed in a refrigerator at 4 °C for storage. When in experiment, the required amount of liposome solution was mixed with PBS of pH 6.8 and added to a cell culture dish for co incubation.

### Example 10. Treatment effect of pharmaceutical composition of 6-phosphogluconic acid

### Experimental instruments and materials

1. Blood glucose test strips and blood glucose meter, from Eli Lilly and Company, USA;
2. C57 B6/J Female mice, from Nanjing Gem Pharmaceutical Co., Ltd.;
3. Streptozotocin, from Sigma-Aldrich Company, USA;
4. Ultrasensitive insulin kit, from ALPCO Company, USA;
5. Insulin antibody, from DAKO Company, USA;
6. Glucagon antibody, from ABCAM Company, USA; and
6. Other reagents required for immunofluorescence assay were purchased from Shanghai Sangon Technology Co., Ltd..

### Construction of type 1 diabetes mellitus mouse model

An 8-week-old C57B6/J female mouse was administered intraperitoneally with 200 mg/kg of streptozotocin solution. After 5 days of injection, the random blood glucose level of the mice was measured, mice with blood glucose levels higher than 20 mmol/L were selected and randomly divided into two groups for subsequent experiments.

### Verification of the Treatment Effect of the Derivatives of 6-Phosphogluconic Acid on Type 1 Diabetes Mellitus

Type 1 diabetes mellitus mouse models were administered 200 mg/kg of 6-phosphogluconic acid methyl ester (compound 1-1) or physiological saline at 8 a.m. and 8 p.m. everyday. The random blood glucose level of each mouse was measured once a day. Before the end of the experiment, the mice were fasted for 16 hours, and a small amount of peripheral blood was taken from the tail vein. As illustrated in Fig. 4, compared with the normal saline group, the random blood glucose level of type 1 diabetes mellitus mice treated with 6-phosphogluconic acid methyl ester was significantly reduced.

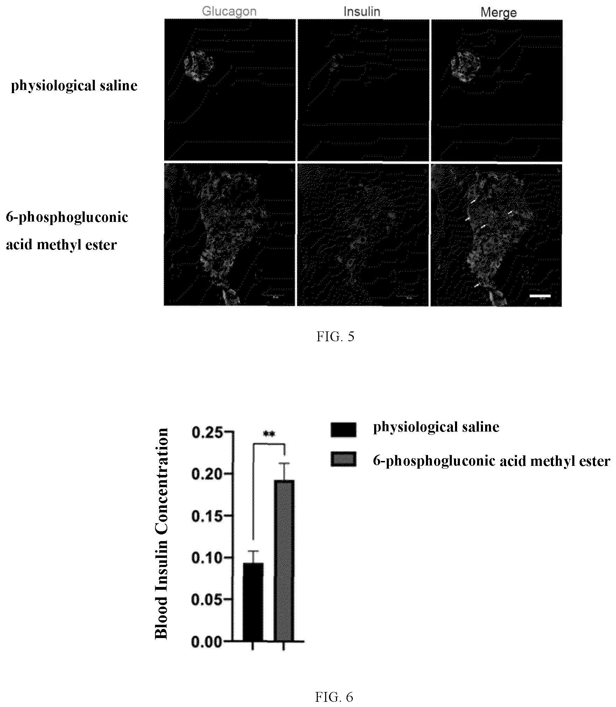

Immunofluorescence assay was used to measure the regeneration of islet β-cells of type 1 diabetes mellitus mice. As illustrated in Fig. 5, compared with the normal saline group, the treatment of 6-phosphogluconic acid methyl ester significantly increased the number of islet β-cells and caused regeneration.

### Validation of the Derivatives of 6-Phosphogluconic Acid in improving insulin deficiency

The concentration of insulin in peripheral blood of mice was measured by an ultrasensitive insulin kit. As illustrated in Fig. 6, compared with the normal saline group, the treatment of 6-phosphogluconic acid methyl ester significantly increased the concentration of fasting insulin in type 1 diabetes mellitus mice.

The above examples are only preferred embodiments of the present disclosure. It should be noted that one skilled in the relevant art can also make several improvements and modifications without departing from the principles of the present disclosure, and these improvements and modifications should also be regarded as the protection scope of the present disclosure.

## Claims

1. Use of 6-phosphogluconic acid and derivatives thereof, or pharmaceutically acceptable salts thereof, in preparing medicament for preventing or treating glucose metabolism disorder diseases.

2. The use of 6-phosphogluconic acid and derivatives thereof, or pharmaceutically acceptable salts thereof, in preparing medicament for preventing or treating glucose metabolism disorder diseases according to claim 1, **characterized in that** the derivative of the 6-phosphogluconic acid is a derivative comprising one or more modified terminal phosphate groups including phosphate esters, phosphines, phosphorane ylides, phosphates, aminophosphate esters, phosphonates, hypophosphites, phosphine oxides, thiophosphates, fluorophosphates, phosphoric anhydrides, bisphosphonates, phosphite esters, phosphonites, thiophosphite esters, dithiophosphate esters and phosphonamides; or a derivative comprising one or more modified terminal carboxylic acid groups including carboxylic esters, aminocarboxylic esters, amides and thiocarbamate esters;
or a derivative comprising one or more modified hydroxyl groups on a sugar chain, which includes substituted hydroxyls, hydroxy esters, hydroxy amides, thiols, substituted thiols and thioesters;
or a derivative in which each structural fragment is modified by a combination of groups mentioned above.

3. The use of 6-phosphogluconic acid and derivatives thereof, or pharmaceutically acceptable salts thereof, in preparing medicament for preventing or treating glucose metabolism disorder diseases according to claim 2, **characterized in that** the derivative of the 6-phosphogluconic acid is represented by Formula (I), (II), (III), (IV) or (V), 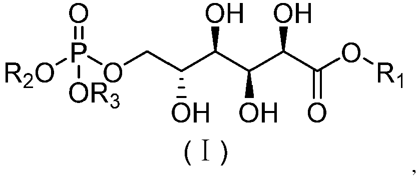 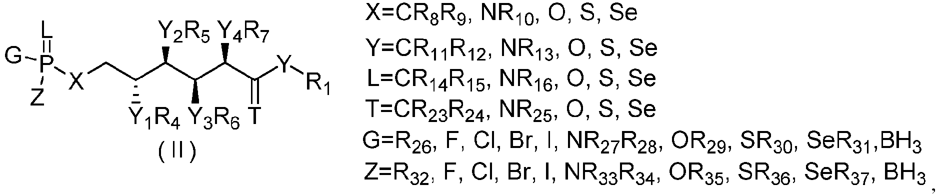 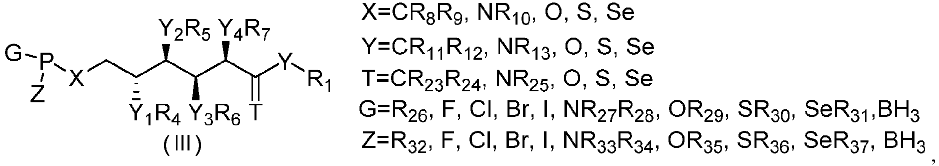 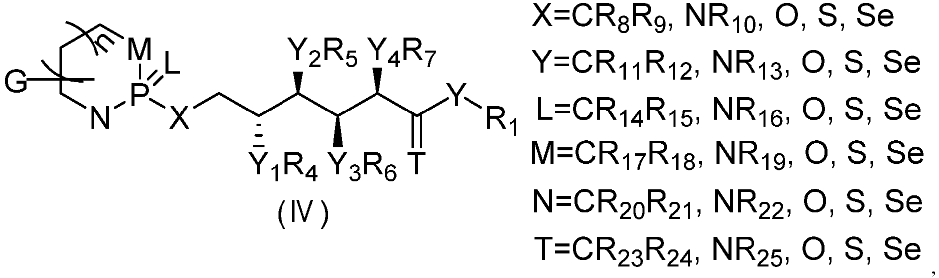 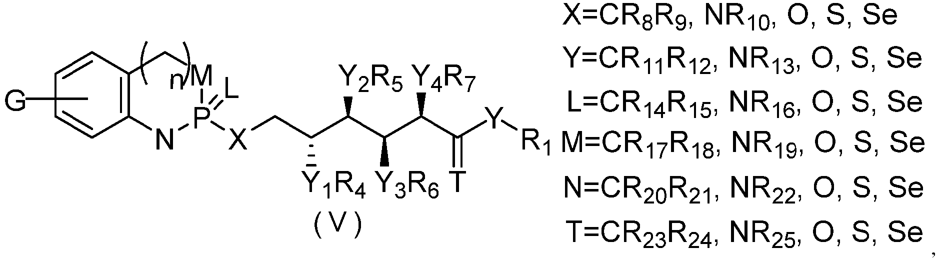 wherein,
R₁₋₃₇ are selected from hydrogen atom, alkyl or substituted alkyl containing less than 10 carbon atoms, cycloalkyl or substituted cycloalkyl, alkylaryl or substituted alkylaryl, alkenyl or substituted alkenyl containing less than 10 carbon atoms, cycloalkenyl or substituted cycloalkenyl, alkenylaryl or substituted alkenylaryl, alkynyl or substituted alkynyl containing less than 10 carbon atoms, alkynylaryl or substituted alkynylaryl containing less than 10 carbon atoms; or selected from biomolecular fragments including oligopeptide, glycoside, protein, nucleotide and fatty acid; or combinations of the above groups and fragments; and wherein a substituent is selected from halogens and heteroatoms.

4. The use of 6-phosphogluconic acid and derivatives thereof, or pharmaceutically acceptable salts thereof, in preparing medicament for preventing or treating glucose metabolism disorder diseases according to claim 1, **characterized in that** the glucose metabolism disorder diseases include diabetes, diabetic ketoacidosis, hyperosmolar hyperglycemic nonketotic coma, diabetic retinopathy, diabetic nephropathy, diabetic foot, atherosclerosis, cerebrovascular accident, peripheral vascular disease, fatty liver disease, malignant tumor, Alzheimer's disease and Parkinson's disease.

5. A derivative of 6-phosphogluconic acid, **characterized in that** the derivative of the 6-phosphogluconic acid is represented by Formula (I), (II), (III), (IV) or (V), 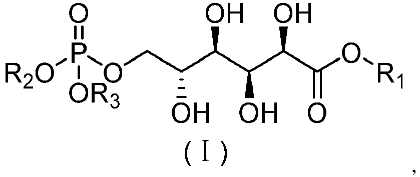 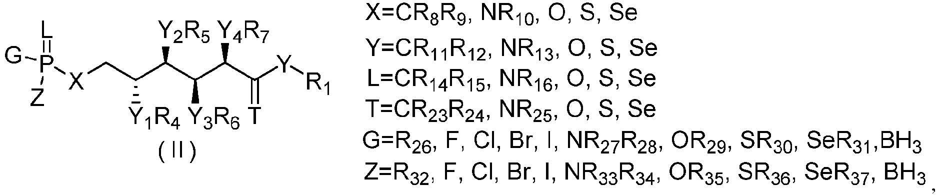 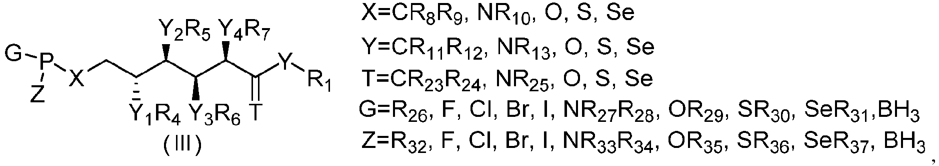 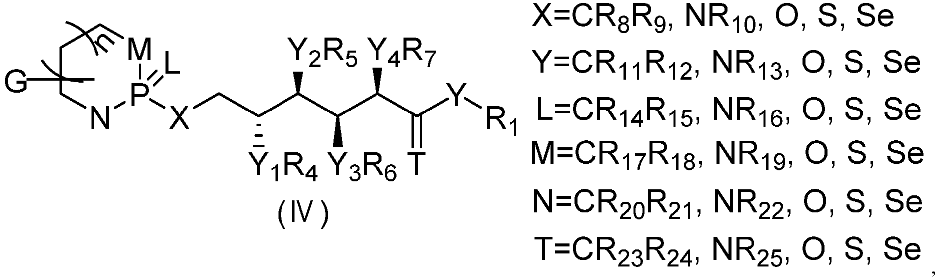 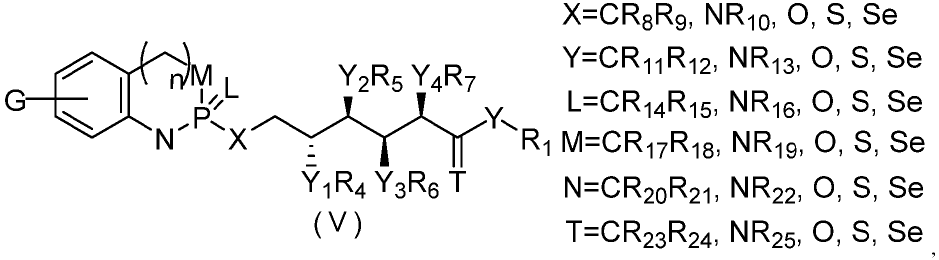 wherein,
R₁₋₃₇ are selected from hydrogen atom, alkyl or substituted alkyl containing less than 10 carbon atoms, cycloalkyl or substituted cycloalkyl, alkylaryl or substituted alkylaryl, alkenyl or substituted alkenyl containing less than 10 carbon atoms, cycloalkenyl or substituted cycloalkenyl, alkenylaryl or substituted alkenylaryl, alkynyl or substituted alkynyl containing less than 10 carbon atoms, alkynylaryl or substituted alkynylaryl containing less than 10 carbon atoms; or selected from biomolecular fragments including oligopeptide, glycoside, protein, nucleotide and fatty acid; or combinations of the above groups and fragments; and wherein a substituent is selected from halogens and heteroatoms.

6. A pharmaceutical composition, **characterized in that** the pharmaceutical composition comprises 6-phosphogluconic acid or derivatives or pharmaceutically acceptable salts thereof, and a pharmaceutically acceptable drug delivery vehicle, wherein the pharmaceutical composition is modified by the drug delivery vehicle to penetrate cell membranes.

7. The pharmaceutical composition according to claim 6, **characterized in that** the derivative of the 6-phosphogluconic acid is a derivative comprises one or more modified terminal phosphate groups including phosphate esters, phosphines, phosphorane ylides, phosphates, aminophosphate esters, phosphonates, hypophosphites, phosphine oxides, thiophosphates, fluorophosphates, phosphoric anhydrides, bisphosphonates, phosphite esters, phosphonites, thiophosphite esters, dithiophosphate esters and phosphonamides;
or a derivative comprising one or more modified terminal carboxylic acid groups including carboxylic esters, aminocarboxylic esters, amides and thiocarbamate esters;
or a derivative comprising one or more modified hydroxyl groups on a sugar chain, which includes substituted hydroxyls, hydroxy esters, hydroxy amides, thiols, substituted thiols and thioesters;
or derivatives in which each structural fragment is modified by a combination of groups mentioned above.

8. The pharmaceutical composition according to claim 7, **characterized in that** the derivative of the 6-phosphogluconic acid is represented by Formula (I), (II), (III), (IV) or (V), 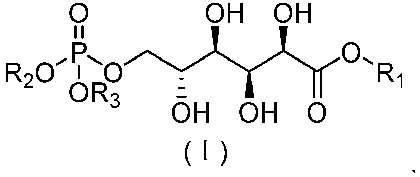 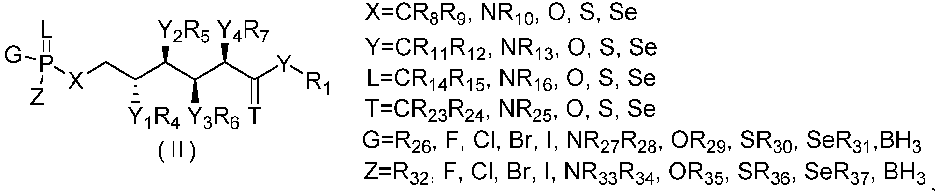 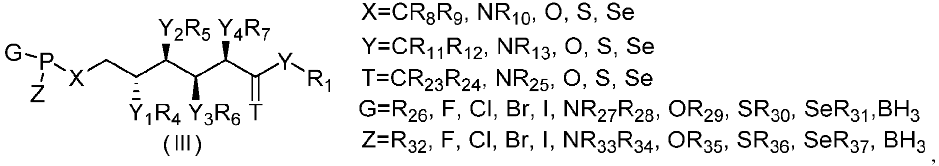 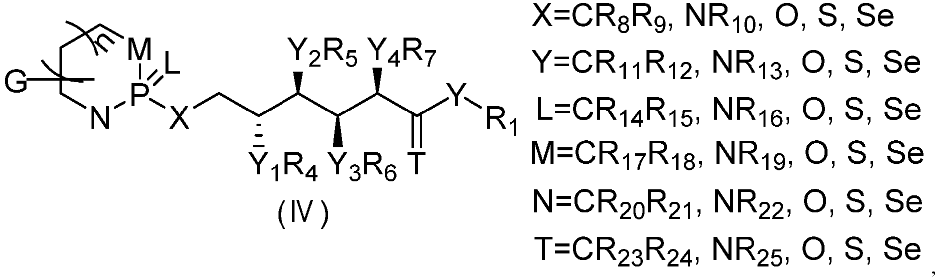 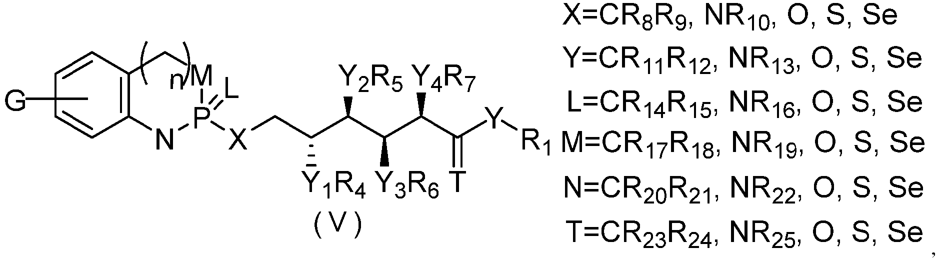 wherein,
R₁₋₃₇ are selected from hydrogen atom, alkyl or substituted alkyl containing less than 10 carbon atoms, cycloalkyl or substituted cycloalkyl, alkylaryl or substituted alkylaryl, alkenyl or substituted alkenyl containing less than 10 carbon atoms, cycloalkenyl or substituted cycloalkenyl, alkenylaryl or substituted alkenylaryl, alkynyl or substituted alkynyl containing less than 10 carbon atoms, alkynylaryl or substituted alkynylaryl containing less than 10 carbon atoms; or selected from biomolecular fragments including oligopeptide, glycoside, protein, nucleotide and fatty acid, or combinations of the above groups and fragments; and wherein a substituent is selected from halogens and heteroatoms.

9. The pharmaceutical composition according to claim 6, **characterized in that** the drug delivery vehicle is a nano drug delivery system including liposomes, polymeric micelles or microcapsules, lipid nanoparticles, and chitosan nanoparticles, wherein the 6-phosphogluconic acid or derivatives or pharmaceutically acceptable salts thereof are encapsulated in the drug delivery vehicle.

10. Use of the pharmaceutical composition according to any one of claims 6 to 9 in preparing medicament for preventing or treating glucose metabolism disorder diseases, **characterized in that** the glucose metabolism disorder diseases include diabetes, diabetic ketoacidosis, hyperosmolar hyperglycemic nonketotic coma, diabetic retinopathy, diabetic nephropathy, diabetic foot, atherosclerosis, cerebrovascular accident, peripheral vascular disease, fatty liver disease, malignant tumor, Alzheimer's disease and Parkinson's disease .
